# EUROPÄISCHE PATENTSCHRIFT

(11) **EP 3 351 192 B1**
(45) Veröffentlichungstag und Bekanntmachung des Hinweises auf die Patenterteilung: **18.09.2024**
(21) Anmeldenummer: 18152176.6
(22) Anmeldetag: 17.01.2018
(51) Int. Cl.: A61B 17/29, A61B 17/00

(54) **CHIRURGISCHES INSTRUMENT, INSBESONDERE FÜR DIE NEUROCHIRURGIE**
SURGICAL INSTRUMENT, IN PARTICULAR FOR NEUROSURGERY
INSTRUMENT CHIRURGICAL, EN PARTICULIER POUR LA NEUROCHIRURGIE

(30) Priorität: 20.01.2017 DE 102017101093
(43) Veröffentlichungstag der Anmeldung: 25.07.2018
(73) Patentinhaber: Karl Storz SE & Co. KG, 78532 Tuttlingen (DE)
(72) Erfinder: Doll, Frank, 78532 Tuttlingen (DE); Thouément, Yann, 78690 Les Essarts le Roi (FR); Besse, Régis, 78610 Le Perray-en-Yvelines (FR); Kärcher, Daniel, 78532 Tuttlingen (DE); Pfeffer, Marian, 78532 Tuttlingen (DE)
(74) Vertreter: Witte, Weller & Partner Patentanwälte mbB

(56) Entgegenhaltungen:
- EP-A1- 0 694 289
- EP-A2- 2 636 380
- WO-A1-2010/002904
- WO-A1-2016/015233
- WO-A1-2016/061291
- WO-A1-96/36289
- DE-A1- 102006 040 529
- DE-A1- 102008 048 615
- US-A- 5 618 306
- US-A- 6 083 150
- US-A1- 2006 079 933
- US-A1- 2009 171 147
- US-A1- 2012 197 190
- US-A1- 2014 263 554
- US-A1- 2016 100 851

## Beschreibung

Die vorliegende Offenbarung betrifft ein chirurgisches Instrument, insbesondere ein Instrument, das für die Neurochirurgie nutzbar ist. Ein solches Instrument kann auch als neurochirurgisches Instrument bezeichnet werden. Ein weiteres Anwendungsgebiet für derartige Instrumente ist das Gebiet der Hirnchirurgie. Die Begriffe "Hirnchirurgie" und "Neurochirurgie" haben einen sich zumindest teilweise überlappenden Bedeutungsgehalt.

Aus der US 2014/263554 A1 ist ein chirurgisches Klammergerät bekannt, mit einem Griff, einem Zündelement, und einem Elektromotor, wobei der Elektromotor das Zündelement während eines ersten Betriebszustands vorschiebt, das Zündelement während eines zweiten Betriebszustands einzieht und während eines dritten Betriebszustands eine Rückmeldung an den Griff überträgt.

Aus der US 2009/171147 A1 ist ein chirurgisches Instrument bekannt, mit einem Instrumentenschaft mit einem proximalen und einem distalen Ende, einem Werkzeug, das am distalen Ende des Instrumentenschafts angeordnet ist, einem Bediengriff, der mit dem proximalen Ende des Instrumentenschafts gekoppelt ist, einem distalem Bewegungselement zum Koppeln des distalen Endes des Instrumentenschafts mit dem Werkzeug, einem proximalen Bewegungselement zur Kopplung des proximalen Endes des Instrumentenschafts mit dem Handgriff, einem Betätigungsmittel, das sich zwischen dem distalen und dem proximalen Bewegungselement erstreckt, um diese zu koppeln, um das Werkzeug zu steuern, einer Schaftkupplung am proximalen Bewegungselement, die selektiv mit einem distalen Empfängerabschnitt des Bediengriffs koppelbar ist, und mit einem Betätigungskabel, das sich von der Schaftkupplung zu dem Werkzeug erstreckt, um die Betätigung des Werkzeugs zu steuern.

Allgemein betrifft die vorliegende Offenbarung Instrumente auf dem Gebiet der minimal-invasiven Chirurgie. In diesem Zusammenhang ist anzumerken, dass im Bereich der Neurochirurgie und/oder der Hirnchirurgie sehr hohe Anforderungen an die Kompaktheit des Instruments, die Führungsgenauigkeit, die Bedienbarkeit sowie die Präzision allgemein bestehen. Die vorstehend beschriebenen Anforderungen gelten grundsätzlich auch für chirurgische Instrumente allgemeiner Art, wie etwa endoskopische und/oder laparoskopische Instrumente. Jedoch gelten für neurochirurgische Instrument und/oder gehirnchirurgische Instrumente noch andere Maßstäbe und Anforderungen. Es hat sich gezeigt, dass Konstruktionsprinzipien, die bei chirurgischen Instrumenten und invasiven Instrumenten allgemeiner Art umsetzbar sind, nicht direkt auf neurochirurgische und/oder hirnchirurgische Instrumente übertragbar sind.

Dies schließt nicht aus, dass Instrumente im Sinne der vorliegenden Offenbarung für die HNO-Chirurgie (Hals-Nasen-Ohren Chirurgie) und andere klassische Bereiche der Chirurgie nutzbar sind. Allgemein können derartige Instrumente dann genutzt werden, wenn ein geringer Platzbedarf und eine gewisse Flexibilität gefordert ist. Instrumente gemäß der vorliegenden Offenbarung können als Miniatur-Instrumente gestaltet sein, insbesondere im Hinblick auf ihren Schaftdurchmesser.

Im Sinne der vorliegenden Offenbarung ist eine proximale Seite eine vom Patienten abgewandte und einem Operateur zugewandte Seite des Instruments. Demgemäß ist eine distale Seite eine dem Patienten zugewandte und vom Operateur abgewandte Seite des Instruments. Insbesondere auf dem Gebiet der invasiven Chirurgie wird das distale Ende des Instruments in das Körperinnere des Patienten eingeführt. Üblicherweise wird das Instrument am proximalen Ende gehalten bzw. geführt, wobei auch Ausführungsformen vorstellbar sind, bei denen sich das proximale Ende des Instruments über den Handhabungsabschnitt hinaus erstreckt.

Aus der US 2016/0262738 A1 ist ein chirurgisches Instrument allgemeiner Art bekannt, welches einen Schaft umfasst, an dessen distalem Ende ein auslenkbarer Arm angeordnet ist, und an dessen proximalen Ende eine Handhabe zur Betätigung des auslenkbaren Arms vorgesehen ist. Die Handhabe ist über zwei Drähte mit dem auslenkbaren Arm zu dessen Betätigung verbunden. Jeweils einer der beiden Drähte wird nach proximal gezogen, um den auslenkbaren Arm wechselseitig zu Verschwenken. Es handelt sich bei dem Instrument gemäß der US 2016/0262738 A1 nicht explizit um ein neurochirurgisches Instrument.

Aus der US 2016/0113732 A1 ist ein chirurgisches Instrument allgemeiner Art bekannt, mit einer distalen Schaftbaugruppe und einem proximalen Griffgehäuse. Zwischen der Schaftbaugruppe und dem Griffgehäuse ist eine Schnittstelle vorgesehen, so dass die Schaftbaugruppe und das Griffgehäuse voneinander lösbar sind. Ferner weist das Instrument an seinem distalen Ende ein Greifwerkzeug mit zwei Maulteilen auf, welche über Zugkabel Also durch Zugbewegungen, geöffnet werden können. Es handelt sich bei dem Instrument gemäß der US 2016/0113732 A1 nicht explizit um ein neurochirurgisches Instrument.

Aus der WO 2016/061291 A1 ist ein chirurgisches Instrument allgemeiner Art bekannt, welches einen Betätigungshebel umfasst, der eine Schwenkachse aufweist, die quer zur Längserstreckung des Instruments orientiert ist, wobei der Betätigungshebel ein Gehäuse am proximalen Ende des Instruments zumindest teilweise seitlich umgreift. Der Betätigungshebel weist Arme auf, die in Nuten eines am Instrument aufgenommenen Rings eingreifen, um den Ring nach proximal oder nach distal zu bewegen. Auf diese Weise kann ein distaler Abschnitt eines Schafts des Instruments ausgelenkt werden. Es handelt sich bei dem Instrument gemäß der US 2016/0113732 A1 nicht explizit um ein neurochirurgisches Instrument.

Aus der JP 2015-198881 A ist ein chirurgisches Instrument bekannt, welches auch für die Neurochirurgie verwendbar sein soll. Das Instrument weist an seinem distalen Ende einen Maulkopfmit zwei Maulteilen auf, die über eine Stange betätigt werden können. Bei der Stange handelt es sich um eine hohe gestaltete Saugstange. Ein Zug an der Stange nach proximal schließt die beiden Maulteile. Ein Schub an der Stange nach distal öffne die beiden Maulteile, wobei ferner eine distale Saugöffnung der Stange nach distal geschoben wird, um Sekret oder dergleichen abzusaugen.

Aus der WO 96/36289 A1 ist ein neurochirurgisches Instrument mit kleinem Schaftdurchmesser bekannt, wobei das Instrument mit einem Tubus mit einem proximalen Ende und einem distalen Ende, einem flexiblen Draht, der sich durch den Tubus erstreckt, wobei der Draht ein proximales Ende und ein distales Ende aufweist und relativ zum Tubus axial beweglich ist, einem manuelles Betätigungsmittel, das mit den proximalen Enden des Tubus und des Drahtes verbunden ist, um den Tubus und Draht relativ zueinander zu versetzen, einem mechanisch mit dem distalen Ende des Tubus gekoppelten ersten Endeffektor, und mit einem mechanisch mit dem distalen Ende des Drahtes gekoppelten und mit dem ersten Endeffektor drehbar gekoppelten zweiten Endeffektor versehen ist, wobei ein gekrümmter Führungskanal entweder in einem proximalen Abschnitt des ersten Endeffektors oder einem distalen Abschnitt des Tubus vorgesehen ist, und wobei sich der axial versetzbare flexible Draht durch den Führungskanal erstreckt und in diesem geführt ist, um sich sowohl radial als auch axial zu bewegen, wenn das manuelle Betätigungsmittel den Tubus und den Draht relativ zueinander axial versetzt.

Eine neurochirurgische oder hirnchirurgische Behandlung umfasst bspw. das Setzen eines Zugangs an der Schädeldecke eines Patienten. Beispielhaft wird eine Bohrung in die Schädeldecke eingebracht, durch die ein Instrument in den Schädel einführbar ist. Bekannte neurochirurgische Instrumente, vergleiche die obige WO 96/36289 A1, weisen einen geraden Schaft auf, an dessen distalen Ende ein Effektor, etwa in Form einer Zange oder Klemme, ausgebildet ist. Der Schaft ist nicht flexibel und nicht auslenkbar.

Die Zugänge in das Körperinnere des Patienten, insbesondere in den Kopf und/oder den Wirbelsäulenbereich, können nicht beliebig groß ausgeführt werden. Das Ziel ist es, Traumata für den Patienten möglichst zu minimieren. Diese Konstellation kann jedoch dazu führen, dass das Instrument einen gewünschten Zielort im Körperinneren, etwa im Kopf des Patienten, schlichtweg nicht erreichen kann, da der Schaft als Ganzes nicht beliebig verschwenkbar ist. Dies würde unter Umständen dazu führen, dass ein weiterer Zugang hergestellt werden muss.

In anderen Bereichen der Chirurgie, etwa auf dem Gebiet der laparoskopischen Chirurgie, sind abwinkelbare Instrumente bekannt. Solche Instrumente weisen Schwenkmechanismen und/oder Auslenkmechanismen auf, die etwa am Schaft selbst oder am distalen Ende des Schaftes ausgebildet sind. Auf diese Weise kann ein zusätzlicher Bewegungsfreiheitsgrad für das Instrument bereitgestellt werden. Beispielhaft kann eine am distalen Ende des Instruments aufgenommene Zange insgesamt verschwenkt werden.

Auf diese Weise können etwa Bereiche im Körperinneren erreicht werden, die seitlich von einer gedachten Hauptachse des Schaftes beabstandet sind. Wie vorstehend bereits ausgeführt, weisen jedoch konventionelle endoskopische Instrumente und/oder laparoskopische Instrumente grundsätzlich größere Abmessungen auf, als neurochirurgische Instrumente. Beispielsweise kann ein Schaftdurchmesser eines laparoskopischen Instruments 10 mm, 12 mm, oder sogar noch mehr betragen. Der Schaftdurchmesser eines neurochirurgischen Instruments, das etwa auf dem Gebiet der Hirnchirurgie genutzt wird, ist deutlich kleiner. Hier kann ein Schaftdurchmesser etwa maximal 3,5 mm, vorzugsweise maximal 3,0, mm, weiter bevorzugt etwa 2,7 mm oder gar weniger betragen. Schon aus diesem Grund können grundsätzlich bekannte Konstruktionsprinzipien für auslenkbare und/oder abwinkelbare Instrumente nicht ohne weiteres auf das Gebiet der Neurochirurgie übertragen werden.

Eine weitere Anforderung, die für neurochirurgische und/oder hirnchirurgische Instrumente im Besonderen gilt, ist die Genauigkeit der Bewegung und/oder die Positionierbarkeit, Positioniergenauigkeit, sowie die Positionierwiederholbarkeit.

Insbesondere bei Operationen, die im Gehirn des Patienten vorgenommen werden, können kleinste Abweichungen und Ungenauigkeiten zu Beschädigungen des benachbarten Gewebes führen. Auch hier gilt, dass sich Gestaltungsprinzipien bekannter abwinkelbarer Instrumente aus dem Bereich der laparoskopischen Chirurgie nicht ohne weiteres auf den Bereich der Neurochirurgie übertragen lassen.

Effektive Wege und Bewegungen, die das Instrument bspw. im Schädel des Patienten ausführt, sind sehr klein. Dies bedingt eine ausgeprägte Feinfühligkeit und Führungsgenauigkeit der Instrumente und der daran/darin verbauten Mechanismen. Bei neurochirurgischen Instrumenten hat die Ergonomie und Handhabbarkeit eine sehr hohe Bedeutung. Das Instrument soll feinfühlig und hochgenau positioniert werden können. Die Positionierung soll mit hoher Wiederholgenauigkeit erfolgen.

Grundsätzlich ist es vorstellbar, neurochirurgische Instrumente mit nicht auslenkbaren/abwinkelbaren Schäften zu nutzen. Jedoch weisen derartige Instrumente nur einen sehr begrenzten Aktionsradius auf. Es gibt Extremfälle, bei denen Instrumente mit geraden Schäften das Arbeitsgebiet schlichtweg nicht erreichen können, da kein geeigneter Zugang (etwa Bohrung in die Schädeldecke) platziert werden kann.

Ferner besteht der Wunsch nach zerlegbaren Instrumenten auch auf dem Gebiet der Neurochirurgie. Ein zerlegbares Instrument weist zumindest eine Schnittstelle auf, an der Baugruppen des Instruments definiert voneinander lösbar sind. Dies kann bspw. eine Schaftbaugruppe und ein Griffstück des Instruments betreffen. Es hat sich gezeigt, dass die Gestaltung zerlegbarer Instrumente häufig zu einer Verringerung der Genauigkeit führt. Mit anderen Worten ist zu beobachten, dass entsprechende Schnittstellen, die für die Zerlegbarkeit der Instrumente erforderlich sind, häufig einen Anstieg des mechanischen Spiels und folglich eine Verringerung der Führungsgenauigkeit bedingen.

Ferner besteht bei chirurgischen Instrumenten, die abwinkelbar/auslenkbar gestaltet sind, häufig eine Wechselwirkung zwischen den beteiligten Freiheitsgraden. Mit anderen Worten kann bspw. eine Betätigung eines Effektors (etwa Öffnen und Schließen einer Zange) gewisse Auswirkungen auf einen Mechanismus für die Auslenkung/Abwinkelfunktion des Instruments haben. Auch dies kann grundsätzlich zu einer Verringerung der Genauigkeit und zu einer Erhöhung des Spiels im System führen.

Vor diesem Hintergrund liegt der vorliegenden Offenbarung die Aufgabe zugrunde, ein chirurgisches Instrument, insbesondere ein für die Neurochirurgie und/oder Hirnchirurgie geeignetes Instrument, anzugeben, das mit hoher Genauigkeit und Präzision bedienbar ist. Freiheitsgrade des Instruments sollen präzise und mit hoher Wiederholgenauigkeit steuerbar sein. Das Instrument ist möglichst mit nur einem geringen Spiel in den beteiligten Bewegungsmechanismen für die Freiheitsgrade versehen. Vorzugsweise ist das Instrument zerlegbar gestaltet, wobei sich ergebende Schnittstellen keine nachteiligen Auswirkungen auf die Genauigkeit bzw. das Spiel im System mit sich bringen. Es ist weiter bevorzugt, wenn das Instrument einen erweiterten Operationsradius bzw. ein erweitertes Operationsgebiet aufweist, so dass auch Gewebebereiche erreichbar sind, die bei gesetztem Instrument von einer Hauptachse des Schaftes beabstandet sind. Das Instrument soll eine spielarme oder gar spielfreie Ansteuerung sowohl des Effektors als auch einer Winkelposition/Auslenkposition des Effektors als Ganzes in Bezug auf den Schaft ermöglichen.

Gemäß einem ersten Aspekt der vorliegenden Offenbarung wird die Aufgabe der Erfindung durch ein chirurgisches Instrument, insbesondere ein Instrument für die Neurochirurgie, gelöst, das Folgendes aufweist:
- einen Schaft, wobei am Schaft ein Gelenkabschnitt ausgebildet ist, der vorzugsweise ausgehend von einer Mittelposition einseitig auslenkbar ist,
- ein Lagerstück mit einem sich von einem distalen zu einem proximalen Ende erstreckenden Durchgang zur Aufnahme eines zumindest proximalen Abschnittes des Schafts,
- einen proximalen Handhabungsabschnitt an einem proximalen Ende des Schaftes,
- einen distalen Effektor an einem distalen Ende des Schaftes, und
- eine Schnittstelle, an der das Instrument in einen distalen und einen proximalen Teil zerlegbar ist, wobei das Instrument an der Schnittstelle in eine distale Schaftbaugruppe und ein proximales Griffstück zerlegbar ist, wobei der Effektor über einen Betätigungsmechanismus gesteuert wird, der ein Schubstück, insbesondere eine Schubstange oder einen Schubdraht, aufweist,
   wobei der Effektor ein erstes Maulteil und ein zweites Maulteil aufweist, die relativ zueinander verschwenkbar sind,
   wobei das Schubstück nach distal verfahrbar ist, um das erste Maulteil und das zweite Maulteil aufeinander zu zu bewegen und zu schließen, wobei die Schnittstelle auf der Seite der Schaftbaugruppe ein Druckstück sowie auf der Seite des Griffstücks einen Schieber umfasst, der auf das Druckstück aufsteckbar ist, das mit dem Schieber zur Krafteinleitung in das Druckstück koppelbar ist,
   wobei das Druckstück am proximalen Ende des Schubstücks vorgesehen ist, wobei das Schubstück durch eine Feder nach proximal vorgespannt ist, die sich zwischen dem Druckstück und einem distalen Anschlag am Lagerstück erstreckt, um das Schubstück in Richtung auf das proximale Ende zu drängen, und
   wobei der Schieber dazu ausgebildet ist, eine Schiebebewegung auf das Druckstück zu übertragen, um das Schubstück in Richtung auf das distale Ende des Instruments zu verlagern.

Auf diese Weise wird die Aufgabe der Erfindung vollständig gelöst.

Erfindungsgemäß erlaubt nämlich die Schnittstelle in einfacher Weise eine Trennung bzw. ein Zerlegen des Instruments. Dies kann bspw. die Herstellung, die Montage, die Handhabung, die Wartung, die Reparatur und/oder die Reinigung des Instruments deutlich vereinfachen.

Die Gestaltung gemäß dem vorstehend beschriebenen Aspekt kann grundsätzlich mit Gestaltungen gemäß anderen der hierin beschriebenen Aspekte kombiniert werden. Gleichwohl stellt diese Gestaltung auch in Alleinstellung eine Offenbarung dar, die Gegenstand unabhängiger Ansprüche sein kann. Demgemäß kann der Gegenstand gemäß dem vorstehenden Aspekt im Rahmen dieser Anmeldung und/oder im Rahmen von Teilanmeldungen in Alleinstellung und/oder beliebiger Kombination mit Gestaltungen gemäß den anderen Aspekten verfolgt werden.

Die Bezeichnungen "Schaftbaugruppe" und "Griffstück" sollen nicht in einschränkender Weise verstanden werden. Beispielhaft schließt die Bezeichnung "Griffstück" nicht aus, dass auch bei der Schaftbaugruppe Griffelemente, Betätigungselemente oder dergleichen ausgebildet sind. Allgemein beschriebt der Begriff "Schaftbaugruppe" einen distalen Teil des Instruments. Allgemein beschreibt der Begriff "Griffstück" einen proximalen Teil des Instruments. Beispielsweise ist es vorstellbar, den Auslenkmechanismus für den Gelenkabschnitt der Schaftbaugruppe zuzuordnen. Ferner ist es vorstellbar, einen Betätigungsmechanismus für den Effektor zumindest teilweise beim Griffstück anzuordnen.

Vorzugsweise erlaubt die Gestaltung der Schnittstelle eine spielarme oder sogar spielfreie Kopplung. Insbesondere erlaubt die Gestaltung der Schnittstelle vorzugsweise eine klapperfreie Kopplung der Schaftbaugruppe und des Griffstücks.

Gemäß einer weiteren beispielhaften Ausgestaltung des Instruments umfasst die Schnittstelle Passteile, die ein männliches Teil und ein weibliches Teil umfassen, die miteinander koppelbar sind. Vorzugsweise sind zu Führungszwecken Zylinderflächen vorgesehen, die eine konzentrische Ausrichtung sicherstellen.

Die Passteile können ferner mit Konusflächen versehen sein, wobei zu Führungszwecken vorzugsweise ferner Zylinderflächen vorgesehen sind, die ebenso an den Passteilen ausgebildet sind. Auf diese Weise kann über die Passteile sowohl eine konzentrische Ausrichtung als auch eine axiale Ausrichtung zwischen der Schaftbaugruppe und dem Griffstück erfolgen. Die Zylinderflächen können Führungskräfte übertragen und somit eine Belastung der Konusflächen beim Gebrauch des Instruments reduzieren. Vorzugsweise schließen sich die Zylinderflächen an die Konusflächen an, so dass etwa die Konusfläche des weiblichen Teils als Montagehilfe für die Zylinderfläche des männlichen Teils dient.

Das männliche Teil kann auch als Innenteil bezeichnet werden. Das weibliche Teil kann auch als Außenteil bezeichnet werden. Zumindest eines der beiden Teile ist direkt oder mittelbar mit dem Schaft gekoppelt, wenn das Instrument an der Schnittstelle zerlegt ist. Demgemäß ist das andere der beiden Teile mittelbar oder unmittelbar mit dem Griffstück gekoppelt, wenn das Instrument zerlegt ist.

Gemäß einer weiteren beispielhaften Ausgestaltung des Instruments ist der Schnittstelle ein Verschlussbügel zugeordnet, der dazu ausgebildet ist, an einen Halteabschnitt anzugreifen, um eine axiale Relativlage zwischen der Schaftbaugruppe und dem Griffstück zu sichern. Beispielsweise ist der Verschlussbügel gelenkig am Griffstück aufgenommen. Beispielsweise ist der Halteabschnitt bei der Schaftbaugruppe ausgebildet, insbesondere bei einem Lagerstück der Schaftbaugruppe.

Der Verschlussbügel ist dazu ausgestaltet, den Halteabschnitt zu übergreifen. Der Halteabschnitt ist bspw. als Absatz gestaltet. Beispielsweise kann der Verschlussbügel gelenkig an einem Trägerschaft aufgenommen sein, der dem Griffstück zugeordnet ist. Somit kann auch der Verschlussbügel relativ zu einer Längsachse des Instruments verschwenkt werden. Eine Schwenkachse des Verschlussbügels ist demgemäß in zumindest beispielhaften Ausgestaltungen senkrecht zur Längsachse des Instruments.

Gemäß einer weiteren beispielhaften Ausgestaltung des Instruments ist der Verschlussbügel biegsam gestaltet. Der Verschlussbügel ist hinreichend elastisch gestaltet und demgemäß etwa aus einem Metallwerkstoff, insbesondere aus Edelstahl, gefertigt. Es kann sich um einen Verschlussbügel auf Basis eines Edelstahlblechs handeln. Alternativ ist der Verschlussbügel aus Kunststoff hergestellt. Der Verschlussbügel kann eine spielarme oder spielfreie Lagesicherung für den gefügten Zustand des Instruments bewirken. Der Verschlussbügel verriegelt die Schnittstelle, vorzugsweise spielarm oder spielfrei, insbesondere in einer axialen Richtung entlang der Längserstreckung des Instruments. Somit sichert der Verschlussbügel die durch die Gestaltung der Passteile bewirkte Lagedefinition.

Gemäß einer weiteren beispielhaften Ausgestaltung des Instruments ist am Verschlussbügel ein Halteausleger ausgebildet, der den Halteabschnitt zumindest abschnittsweise umgibt. Beispielsweise umfasst der Halteausleger einen Lagesicherungsabschnitt und einen aufgeweiteten Freigabeabschnitt. Demgemäß vereinfacht die Gestaltung des Verschlussbügels das Öffnen und Verriegeln der Schnittstelle. Ferner verbessert sich die Bedienergonomie. Die Gefahr von Fehlbedienungen kann sich verringern.

Beispielhaft ist der Halteausleger an einem distalen Ende des Verschlussbügels ausgebildet. Der Verschlussbügel ist bspw. am Trägerschaft aufgenommen und erstreckt sich in Richtung auf den Halteabschnitt, der etwa am Lagerstück vorgesehen ist. Durch Verschwenken des Verschlussbügels kann der Halteausleger in einen Eingriff mit dem Halteabschnitt gebracht oder vom Halteabschnitt ausgerückt werden.

Mit anderen Worten ist der Verschlussbügel selbst das "Werkzeug", um die Schnittstelle zu verriegeln oder zu öffnen. Somit erlaubt die Schnittstelle ein werkzeugloses Fügen und Trennen des Instruments.

Der Halteausleger erstreckt sich gemäß einer beispielhaften Ausführungsform im Wesentlichen senkrecht zur Längsachse des Instruments. Der Halteabschnitt und der Freigabeabschnitt können gemeinsam eine Schlüssellochkontur definieren, wobei der Halteabschnitt die Engstelle und der Freigabeabschnitt die aufgeweitete Stelle bildet.

Gemäß einer weiteren beispielhaften Ausgestaltung des Instruments ist der Verschlussbügel in Richtung auf eine Verschlusstellung vorgespannt und durch eine entgegengerichtete Bewegung lösbar. Auf diese Weise wird sichergestellt, dass die Schnittstelle nicht unabsichtlich entriegelt wird.

Gemäß einer weiteren beispielhaften Ausgestaltung des Instruments weist die Schnittstelle ferner eine Drehlagensicherung auf. Die Drehlagensicherung kann beispielhaft einen Führungsstift umfassen, der in eine sich axial erstreckende, zumindest einseitig offene Nut eingreift. Beispielhaft ist der Führungsstift am männlichen Passteil aufgenommen. Demgemäß ist die Nut am weiblichen Passteil ausgebildet. Der Führungsstift kann etwa radial zur Längsachse des Instruments orientiert sein.

Gemäß einer erfindungsgemäßen Ausgestaltung des Instruments umfasst die Schnittstelle ferner eine lösbare Kopplung zwischen einem Druckstück am proximalen Ende eines Schubstücks zur Steuerung des Effektors und einem Schieber zur Krafteinleitung in das Druckstück. Auf diese Weise kann sich die Schnittstelle eben auch über zumindest einen Mechanismus des Instruments erstrecken, wobei vorzugsweise dort auch einfach lösbare Fügeteile vorgesehen sind. In diesem Zusammenhang ist es von Vorteil, wenn der Effektor über ein Schubstück, also über ein auf Druck beanspruchbares Element, gesteuert wird. Demgemäß kann nämlich in einfacher Weise der mit dem Druckstück koppelbare Schieber bei der Montage der Schaftbaugruppe und des Griffstücks aufgeschoben und bei der Demontage abgezogen werden.

Gemäß einem weiteren Aspekt der Offenbarung wird die Aufgabe der Erfindung durch ein chirurgisches Instrument, insbesondere für Instrument für die Neurochirurgie, gelöst, wobei das Instrument Folgendes aufweist:
- einen Schaft,
- einen proximalen Handhabungsabschnitt an einem proximalen Ende des Schaftes, und
- einen distalen Effektor an einem distalen Ende des Schaftes,
wobei der Effektor über einen Betätigungsmechanismus gesteuert wird, der ein Schubstück, insbesondere eine Schubstange oder einen Schubdraht, aufweist.

Auch auf diese Weise wir die Aufgabe der Erfindung vollständig gelöst.

Erfindungsgemäß erlaubt die Ansteuerung des Effektors über ein Schubstück (und nicht über ein Zugstück in Form einer Zugstange oder eines Zugdrahts) eine Betätigung des Effektors, die keine oder nur wenige Auswirkungen auf eine aktuelle Schwenkkonfiguration des Schaftes, insbesondere auf einen aktuellen Schwenkzustand eines Gelenkabschnitts, hat. Dies kann dazu beitragen, das Spiel bei den verschiedenen Freiheitsgraden des Instruments weiter zu minimieren. Ferner können etwaige Rückkopplungen zwischen dem Freiheitsgrad des Effektors und dem Freiheitsgrad des Auslenkmechanismus minimiert oder gar vollends vermieden werden.

Die Gestaltung gemäß dem vorstehend beschriebenen Aspekt kann grundsätzlich mit Gestaltungen gemäß anderen der hierin beschriebenen Aspekte kombiniert werden. Gleichwohl stellt diese Gestaltung auch in Alleinstellung eine Offenbarung dar, die Gegenstand unabhängiger Ansprüche sein kann. Demgemäß kann der Gegenstand gemäß dem vorstehenden Aspekt im Rahmen dieser Anmeldung und/oder im Rahmen von Teilanmeldungen in Alleinstellung und/oder beliebiger Kombination mit Gestaltungen gemäß den anderen Aspekten verfolgt werden.

Gemäß einer beispielhaften Ausgestaltung des Instruments weist der Effektor ein erstes Maulteil und ein zweites Maulteil auf, die relativ zueinander verschwenkbar sind. Dies umfasst Ausgestaltungen, bei denen beide Maulteile aufeinander zu und voneinander weg verschwenkbar sind. Ferner sind jedoch auch Ausgestaltungen vorstellbar, bei denen lediglich ein Maulteil in Bezug auf das andere Maulteil verschwenkbar ist. Beispielhaft können die Maulteile zwischen einer ersten, geschlossenen Stellung und einer zweiten geöffneten Stellung verschwenkt werden.

Der Begriff "Verschwenken" bzw. "Schwenkbewegung" soll nicht einschränkend verstanden werden. Allgemein ist hierunter ein Aufeinanderzubewegen und ein Voneinanderwegbewegen der Maulteile zu verstehen, um diese zwischen der ersten und der zweiten Stellung zu bewegen.

Der Effektor kann bspw. als Greifer, Zange, Schere, Klemme und dergleichen vorstellbar sein. Auch eine Gestaltung als Koagulationsklemme ist vorstellbar. Ferner ist die Gestaltung des Effektors als Kürette, Biopsiezange oder in ähnlicher Weise vorstellbar.

Gemäß einer beispielhaften Ausgestaltung kann der Effektor durch einen Druck auf das Schubstück in Richtung auf das distale Ende des Schaftes aus der zweiten, geöffneten Stellung in die erste, geschlossene Stellung bewegt werden. Demgemäß wird das Schubstück gemäß einer weiteren beispielhaften Ausgestaltung des Instruments nach distal verfahren, um das erste Maulteil und das zweite Maulteil aufeinander zu zu bewegen, insbesondere zu schließen.

Gemäß einer weiteren beispielhaften Ausgestaltung des Instruments ist das Schubstück an seinem distalseitigen Ende mit einen Koppelstück verbunden, das einen ersten Mitnehmer für das erste Maulteil und einen zweiten Mitnehmer für das zweite Maulteil aufweist, wobei das erste Maulteil eine Mitnahmeausnehmung aufweist, wobei das zweite Maulteil eine Mitnahmeausnehmung aufweist, wobei der erste Mitnehmer des Koppelstücks in die Mitnahmeausnehmung des ersten Maulteils eingreift, und wobei der zweite Mitnehmer des Koppelstücks in die Mitnahmeausnehmung des zweiten Maulteils eingreift.

Dies kann Ausgestaltungen umfassen, bei denen die Mitnehmer des Koppelstücks an voneinander abgewandten Seiten des Koppelstücks ausgebildet sind, wobei die Mitnehmer auch in einer Richtung senkrecht zu einer Schwenkrichtung des Effektors sowie senkrecht zu einer Betätigungsrichtung des Schubstücks voneinander beabstandet sind.

Gemäß einer weiteren beispielhaften Ausgestaltung weist das Instrument ferner am distalen Ende des Schaftes ein Kopfstück auf, wobei das erste Maulteil schwenkbar am Kopfstück gelagert ist, wobei das zweite Maulteil schwenkbar am Kopfstück gelagert ist, und wobei Schenkachsen des ersten Maulteils und des zweiten Maulteils parallel zueinander und voneinander beabstandet sind.

Dies kann Ausgestaltungen umfassen, bei denen das Kopfstück am distalen Ende des Gelenkabschnitts angeordnet ist. Das Kopfstück kann insbesondere als Gabelkopf gestaltet sein und ein erstes Seitenteil und ein zweites Seitenteil umfassen, zwischen denen sowohl das Koppelstück als auch das erste Maulteil und das zweite Maulteil aufgenommen sind. Vorzugsweise ist das Koppelstück mittig zwischen dem ersten Maulteil und dem zweiten Maulteil angeordnet. Die Schwenkachsen des ersten Maulteils und des zweiten Maulteils sind beispielhaft jeweils durch einen Vorsprung und eine zugehörige Lagerausnehmung definiert.

Gemäß einer weiteren beispielhaften Ausgestaltung des Instruments weist erste Maulteil eine Führungsbahn auf, wobei das zweite Maulteil eine Führungsbahn aufweist, und wobei ein am Kopfstück aufgenommener Führungsstift in der Führungsbahn des ersten Maulteils und der Führungsbahn des zweiten Maulteils aufgenommen ist. Auf diese Weise kann auch beim Effektor eine Zwangsführung erzeugt werden, die das Spiel im Mechanismus reduziert oder sogar eliminiert. Die Führungsbahnen können wiederum derart gestaltet sein, dass sich eine Zwangsführung für den Führungsstift ergibt.

Beispielhaft ist der Führungsstift parallel zu den Schwenkachsen der beiden Maulteile gestaltet. Der Führungsstift kann in einer Ebene zwischen den Schwenkachsen der beiden Maulteile angeordnet sein.

Gemäß einer weiteren beispielhaften Ausgestaltung des Instruments erstreckt sich das Koppelstück zumindest abschnittsweise zwischen dem ersten Maulteil und dem zweiten Maulteil, wobei der Führungsstift eine Ausnehmung des Koppelstücks durchragt. Demgemäß ist eine zusätzliche Lagesicherung gewährleistet.

Das Koppelstück kann in dem Bereich, in dem es sich zwischen dem ersten Maulteil und dem zweiten Maulteil erstreckt, etwa eine flache Zunge aufweisen, in die eine Längsnut für den Führungsstift eingebracht ist.

Gemäß einer weiteren beispielhaften Ausgestaltung des Instruments ist am proximalen Ende des Schubstücks ein Druckstück vorgesehen, das mit einem Schieber koppelbar ist, wobei der Schieber längsverschieblich am Handhabungsabschnitt gelagert ist, und wobei der Schieber mit einer Handhabe gekoppelt ist, die zumindest einen Betätigungsabschnitt aufweist, der schwenkbar am Handhabungsabschnitt aufgenommen ist. Auf diese Weise ist sowohl der Betätigungsmechanismus am proximalen Ende des Schubstücks als auch der Steuermechanismus am distalen Ende des Schubstücks jeweils als Koppelmechanismus gestaltet. Auf diese Weise kann die Führungsgenauigkeit erhöht werden. Die Handhabe kann etwa zangenartig gestaltet sein, wobei vorzugsweise zwei Betätigungsabschnitte vorgesehen sind, die einen Trägerschaft des Griffstücks zwischen sich einschließen. Demgemäß können die Handhabungsabschnitte zusammengedrückt werden, um den Schieber nach distal zu bewegen, um den Effektor zu schließen.

Gemäß einer weiteren beispielhaften Ausgestaltung des Instruments ist am Handhabungsabschnitt ein Koppelmechanismus ausgebildet, der die Handhabe, den Schieber und zumindest ein Koppelglied umfasst, wobei eine Schwenkbewegung des zumindest einen Betätigungsabschnitts in eine Schubbewegung des Schiebers überführt wird.

Beispielhaft ist der Koppelmechanismus nach Art einer Schubschwinge gestaltet. Sofern die Handhabe zwei Betätigungsabschnitte aufweist, kann der Koppelmechanismus nach Art einer doppelten Schubschwinge gestaltet sein. Dies kann wiederum die Feinfühligkeit und Eindeutigkeit der Bedienung des Effektors erhöhen. Der Koppelmechanismus zur Betätigung des Druckstücks ist Teil des Betätigungsmechanismus für den Effektor.

Gemäß einer weiteren beispielhaften Ausgestaltung des Instruments ist die Handhabe zangenartig gestaltet und mit zwei Armen versehen, die den Betätigungsabschnitten zugeordnet sind, und die in Richtung auf das proximale Ende des Schaftes geöffnet sind, und wobei die Arme den Verschlussbügel und den Steuerhebel zumindest abschnittsweise seitlich umgreifen.

Auf diese Weise kann das Instrument insgesamt eine kompakte Form und eine günstige Ergonomie aufweisen. Die Arme der Handhabe bedecken seitlich zumindest einen wesentlichen Abschnitt des Trägerschafts. Ein weiterer Vorteil der obigen Gestaltung ist das sich für den Operateur des Instruments ergebende günstige Sichtfeld. Dies kann die Steuerung und Betätigung weiter vereinfachen.

Gemäß einer weiteren beispielhaften Ausgestaltung des Instruments sind, entlang einer Haupterstreckungsrichtung des Instruments, von proximal nach distal gesehen, die Handhabe, der Verschlussbügel und der Steuerhebel nacheinander gelenkig aufgenommen sind, wobei Schwenkachsen des Verschlussbügels und des Steuerhebels parallel zueinander sind, wobei eine Schwenkbewegung der Handhabe um eine Schwenkachse erfolgt, die senkrecht zu den Schwenkachsen des Verschlussbügels und des Steuerhebels ist, und wobei vorzugsweise die Handhabe und der Verschlussbügel gelenkig an einem Trägerschaft des Griffstücks aufgenommen sind. Die Schwenkachsen der Handhabe, des Verschlussbügels und des Steuerhebels sind gemäß beispielhaften Ausführungsformen allesamt senkrecht zur Längsachse des Instruments.

Beispielhaft kann zumindest die Handhabe derart gestaltet sein, dass keine eindeutig definierte Schwenkachse vorgesehen ist. Stattdessen ist die Handhabe beispielhaft elastisch verformbar und etwa mit einem Stoffgelenk versehen. Auf diese Weise ergibt sich eine gedachte Schwenkachse für die Handhabe.

Gemäß einer erfindungsgemäßen Ausgestaltung des Instruments ist das Schubstück am proximalen Ende durch eine Feder nach proximal vorgespannt, wobei sich die Feder zwischen dem Druckstück und einem distalen Anschlag am Handhabungsabschnitt erstreckt. Auf diese Weise kann auch das Schubstück permanent einer gewissen Vorspannung unterliegen. Dies kann das Spiel im Betätigungsmechanismus für den Effektor weiter verringern, insbesondere das Spiel bei einer Bewegungsumkehr.

Gemäß einem ersten Aspekt wird die Aufgabe der Erfindung durch ein chirurgisches Instrument, insbesondere ein Instrument für die Neurochirurgie, gelöst, wobei das Instrument Folgendes aufweist:
- einen Schaft, wobei am Schaft ein Gelenkabschnitt ausgebildet ist, insbesondere ein Gelenkabschnitt, der ausgehend von einer Mittelposition einseitig auslenkbar ist,
- einen proximalen Handhabungsabschnitt an einem proximalen Ende des Schaftes,
- einen distalen Effektor an einem distalen Ende des Schaftes, und
- einen Auslenkmechanismus zur Steuerung eines Krümmungszustands des Gelenkabschnitts, der ein erstes Zugelement, insbesondere für eine Auslenkbewegung, sowie ein zweites Zugelement, insbesondere für eine Rückkehrbewegung, aufweist,
wobei das erste Zugelement und das zweite Zugelement, zumindest abschnittsweise während der Bewegung des Gelenkabschnitts, gemeinsam vorgespannt sind.

Auch auf diese Weise wird die Aufgabe vollkommen gelöst.

Erfindungsgemäß erlaubt nämlich die Gestaltung der Zugelemente eine spielarme oder sogar spielfreie Auslenkbewegung bzw. Ablenkung des Gelenkabschnitts. Dies wird dadurch erreicht, dass beide Zugelemente, von denen eigentlich beim Ausschwenken bzw. Einschwenken jeweils nur eines unter Spannung (Zugspannung) gesetzt wird, gemeinsam und richtungsgleich unter Spannung gesetzt werden. Dies bedeutet bspw., dass die Auslenkbewegung weiterhin über einen Zug am ersten Zugelement erfolgt.

Jedoch wird das zweite Zugelement während der Auslenkbewegung zumindest abschnittsweise auch vorgespannt, wenn auch nur mit deutlich geringerer Spannung als das erste Zugelement. Bei der Rückkehrbewegung ist es umgekehrt, das zweite Zugelement wird entsprechend unter Zugspannung gesetzt. Auf das erste Element wird jedoch zumindest abschnittsweise eine Zugspannung aufgebracht, wenn auch mit einem deutlich niedrigerem Spannungsniveau als beim zweiten Zugelement. Der Begriff "abschnittsweise" bezieht sich hierbei bspw. auf einen bestimmten Schwenkwinkelbereich während der Verstellbewegung.

Vorzugsweise ist das Instrument mit einem Gelenkabschnitt versehen, der einseitig auslenkbar bzw. abklappbar ist. Dies hat den Vorteil, dass eine Mittelposition, bei der bspw. der Gelenkabschnitt zu einer Längsachse des Schaftes ausgerichtet ist, einfach mechanisch definiert werden kann, etwa über geeignete Anschläge.

Insgesamt ist der Auslenkmechanismus besonders spielarm, vorzugsweise spielfrei gestaltet. Dies erhöht die Positioniergenauigkeit und die Wiederholbarkeit der Positionierung des Instruments deutlich.

Beispielsweise weisen chirurgische Instrumente, die für die Neurochirurgie und/oder die Hirnchirurgie geeignet sind, Schaftdurchmesser im Bereich von weniger als 3,5 mm, vorzugsweise von weniger als 3,0 mm, weiter bevorzugt von etwa 2,7 mm oder gar weniger auf. Obgleich diese Bauraumrestriktion die gestalterische Freiheit stark limitiert, kann die Spielarmut oder Spielfreiheit über die richtungsgleiche Vorspannung an den Zugelementen bewirkt werden.

Mit anderen Worten wird bspw. eine Zwangssteuerung bzw. eine überbestimmte Führung für das erste Zugelement und das zweite Zugelement bereitgestellt. Wäre dies nicht der Fall, würde bspw. bei der Auslenkbewegung das zweite Zugelement entlastet sein. Bei der Rückkehrbewegung wäre das erste Zugelement entlastet. Das jeweils andere Element wäre auf Zug beansprucht. Dies hätte den Nachteil, dass etwa dann, wenn die Richtung der Verstellbewegung des Gelenkabschnitts geändert wird, ein Spiel im Auslenkmechanismus vorhanden wäre, der zu einer verringerten Positioniergenauigkeit und Wiederholgenauigkeit führen würde.

Da jedoch gemäß dem oben genannten Aspekt beide Zugelemente gemeinsam vorgespannt sind, kann eine solche Bewegungsumkehr spielarm oder sogar spielfrei erfolgen. Dies kann einerseits für Extremlagen des Gelenkabschnitts gelten, etwa für eine vollständig eingefahrene Position (Mittelposition) und/oder eine vollständig ausgefahrene Position (vollständig ausgelenkt) gelten. Gleichwohl ist eine Umkehrbewegung auch an einer Zwischenposition zwischen den Extrempositionen spielarm oder sogar spielfrei realisierbar, wenn am entsprechenden Umkehrpunkt beide Zugelemente richtungsgleich vorgespannt sind. Eine richtungsgleiche Vorspannung liegt etwa dann vor, wenn beide Zugelemente auf Zug vorgespannt sind.

Mit anderen Worten ist der Auslenkmechanismus für die Steuerung des Krümmungszustands zumindest teilweise überdefiniert und/oder überbestimmt gestaltet, um den gewünschten Vorspannungszustand bei den Zugelementen zu bewirken. Bei geeigneter Auslegung muss sich dies nicht nachteilig auf die Betätigungskräfte auswirken. Vielmehr vereinfacht sich die Betätigung, da die spielarme oder spielfreie Gestaltung unmittelbar und direkt auf Steuerbewegungen reagiert.

Gemäß einer beispielhaften Ausgestaltung des Instruments ist der Gelenkabschnitt ausgehend von einer Mittelposition einseitig auslenkbar ist. Gemäß dieser Ausgestaltung ist der Gelenkabschnitt ausgehend von einer Mittelposition nicht in entgegengesetzter Richtung auslenkbar.

Gemäß einer beispielhaften Ausgestaltung des Instruments, ist das erste Zugelement für eine Auslenkbewegung und das zweite Zugelement für eine Rückkehrbewegung vorgesehen.

Gemäß einer beispielhaften Ausgestaltung des Instruments stehen das erste Zugelement und das zweite Zugelement gleichzeitig unter Zugspannung, wenn der Gelenkabschnitt ausgeschwenkt oder eingeschwenkt wird. Dies hat den Vorteil, dass bei einer Bewegungsumkehr nur wenig Spiel oder vorzugsweise gar kein Spiel auftreten kann.

Gemäß einer beispielhaften Ausgestaltung des Instruments sind das erste Zugelement als erster Zugdraht und das zweite Zugelement als zweiter Zugdraht ausgebildet, die auf einander gegenüberliegenden Seiten eines Zentrums des Schaftes angeordnet sind. Auf diese Weise können die Zugelemente zumindest abschnittsweise exzentrisch zur Längsachse/Mittelachse des Schaftes verlaufen. Somit kann ein Kraftangriff an einem der Zugelemente eine Schwenkbewegung des Gelenkabschnitts in Bezug auf den Schaft, insbesondere in Bezug auf ein im Wesentlichen starres Rohr des Schaftes, bewirken.

Bei dem vorstehend beschriebenen Ausführungsbeispiel, das einen einseitig aus einer Mittelposition auslenkbaren Gelenkabschnitt umfasst, kann der erste Zugdraht bspw. als innerer Zugdraht und der zweite Zugdraht bspw. als äußerer Zugdraht bezeichnet werden, wobei sich die Begriffe "innen" und "außen" auf den sich ergebenden Krümmungsradius beim Auslenken beziehen. Beispielsweise sind die Zugelemente in einer gemeinsamen Ebene angeordnet, die ferner die Längsachse des Schaftes schneidet. In dieser Ebene liegen die Zugelemente auf gegenüberliegenden Seiten der Längsachse. Die Zugelemente sind beispielhaft mit einer Effektoraufnahme für den Effektor am distalen Ende des Schaftes gekoppelt. Ein Zug nach proximal an einem der beiden Zugelemente bewirkt entsprechend die Auslenkbewegung oder die Rückkehrbewegung, wobei am jeweils anderen Zugelement zumindest eine leichte Zugspannung angelegt wird. Dies kann das Spiel im Auslenkmechanismus deutlich reduzieren oder sogar eliminieren.

Gemäß einer weiteren beispielhaften Ausgestaltung des Instruments erstrecken sich die Zugelemente jeweils zwischen einem proximalen Koppelpunkt und einem distalen Koppelpunkt im Schaft, wobei die Zugelemente an ihrem proximalen Ende mit einer Steuereinheit gekoppelt sind, die einen schwenkbaren Steuerhebel umfasst. Demgemäß ist der Steuerhebel bspw. um eine Schwenkachse verschwenkbar, die senkrecht zur Längsachse des Schaftes verläuft.

Gemäß einer weiteren Ausgestaltung des Instruments stellt die Steuereinheit eine Zwangssteuerung für das erste Zugelement und das zweite Zugelement bereit. Dies kann bspw. durch die Gestaltung des schwenkbaren Steuerhebels bewirkt werden. Der Steuerhebel kann als Bestandteil eines ebenen Kurvengetriebes gestaltet sein. Eine bewusste überbestimmte und/oder überdefinierte Auslegung des Kurvengetriebes bewirkt eine leichte Vorspannung auf das für die jeweilige Bewegung (Einschwenken/Ausschwenken) eigentlich nicht erforderliche Zugelement.

Die Koppelpunkte der Zugelemente können ebenso in einer Ebene angeordnet sein, die die Längsachse entlang des Schaftes schneidet. Entlang des Schaftes können die Zugelemente zumindest abschnittsweise parallel angeordnet sein.

Die Steuereinheit mit dem schwenkbaren Steuerhebel ist vom distalen Ende des Schaftes deutlich beabstandet. Vorzugsweise ist die Steuereinheit beim proximalen Ende des Schaftes vorgesehen. Dies hat den Vorteil, dass am distalen Ende selbst, an dem der Effektor aufgenommen ist, keine besondere Gestaltung erforderlich ist, um die gewünschte Zugspannung im jeweils anderen Zugelement zu erzeugen, die für die Spielarmut oder Spielfreiheit erforderlich ist.

Gemäß einer weiteren beispielhaften Ausgestaltung des Instruments weist die Steuereinheit am Steuerhebel eine erste Führungsbahn für das erste Zugelement und eine zweite Führungsbahn für das zweite Zugelement auf, wobei am Handhabungsabschnitt eine erste Führungsbahn für das erste Zugelement und eine zweite Führungsbahn für das zweite Zugelement vorgesehen ist. Vorzugsweise sind die erste Führungsbahn und die zweite Führungsbahn am Handhabungsabschnitt zu einer Längsachse des Schaftes geneigt. Vorzugsweise ist das erste Zugelement mit der ersten Führungsbahn am Handhabungsabschnitt und der ersten Führungsbahn des Steuerhebels gekoppelt, wobei das zweite Zugelement mit der zweiten Führungsbahn am Handhabungsabschnitt und der zweiten Führungsbahn des Steuerhebels gekoppelt ist.

Der Steuerhebel ist bspw. schwenkbar an einem Lagerstück aufgenommen, das mit dem Schaft gekoppelt ist. Demgemäß können am Lagerstück die erste Führungsbahn und die zweite Führungsbahn des Handhabungsabschnitts ausgebildet sein.

Beispielsweise sind das erste Zugelement und das zweite Zugelement an ihrem jeweiligen proximalen Ende jeweils mit einem Gleitstück gekoppelt, etwa in Form eines Stiftes. Das Gleitstück für das erste Zugelement ist sowohl in der ersten Führungsbahn am Steuerhebel als auch in der ersten Führungsbahn am Lagerstück aufgenommen. Das zweite Gleitstück ist sowohl in der zweiten Führungsbahn am Steuerhebel als auch in der zweiten Führungsbahn am Lagerstück aufgenommen. Die jeweils erste und zweite Führungsbahn am Steuerhebel sind derart (winklig und radial) zu einer Schwenkachse des Steuerhebels versetzt, das bei der Betätigung des Steuerhebels über dessen Führungsbahnen ein Antrieb der Gleitstücke, also ein Verschieben der Gleitstücke in den am Lagerstück ausgebildeten Führungsbahnen bewirkt wird. Die jeweils ersten Führungsbahnen und zweiten Führungsbahnen der Steuereinheit sind so gestaltet, dass ein Längenversatz, der sich bei der Verstellbewegung des Gelenkabschnitts bei den Zugelementen ergibt, kompensiert wird.

Wie vorstehend bereits ausgeführt, kann das erste Zugelement als inneres Zugelement und das zweite Zugelement als äußeres Zugelement bezeichnet werden. Wenn nun der Gelenkabschnitt um einen bestimmten Winkelbetrag verschwenkt wird, ist der resultierende Weg für das innere Zugelement deutlich kleiner als für das äußere Zugelement.

Gemäß einer weiteren beispielhaften Ausgestaltung des Instruments sind die Führungsbahnen für das erste Zugelement und das zweite Zugelement zur Kompensation eines Längsversatzes ausgebildet, der auf unterschiedliche Biegeradien des ersten Zugelements und des zweiten Zugelements beim Auslenken und Rückkehren des Auslenkmechanismus zurückgeht.

Auf diese Weise wird dem Umstand Rechnung getragen, dass etwa das zweite Zugelement beim Schwenken des Steuerhebels und beim hieraus resultierenden Schwenken des Gelenkabschnitts einen größeren Weg zurücklegt als das erste Zugelement.

Vorzugsweise umfasst die Kompensation des Längenversatzes jedoch keine vollständige Kompensation. Beispielsweise sind die Führungsbahnen derart gestaltet, dass zumindest ein minimaler Längenversatz bzw. Wegversatz beim Schwenken des Steuerhebels verbleibt. Auf diese Weise kann die gewünschte Vorspannung erzeugt werden. Die Steuereinheit kann spielarm oder spielfrei gestaltet sein.

Gemäß einer weiteren beispielhaften Ausgestaltung des Instruments sind die Führungsbahnen derart gestaltet, dass beim Wechsel zwischen zwei Krümmungszuständen beide Zugelemente zumindest abschnittsweise unter Spannung stehen. Vorzugsweise stehen die Zugelemente zumindest beim Wechsel zwischen den beiden Krümmungszuständen unter Zugspannung.

Es ist vorstellbar, beide Zugelemente dauerhaft leicht vorzuspannen, also entlang ihrer Längserstreckung auf Zug zu beanspruchen. Diese Vorspannung kann in jeder Stellung des Gelenkabschnitts gegeben sein. Es versteht sich, dass eine leichte Vorspannung ausreicht, um das Spiel zu minimieren oder gar zur eliminieren. Der Auslenkmechanismus kann reibungsbehaftet ausgelegt sein, so dass sich eine Selbsthemmung ergibt, wenn keine Betätigungskräfte oder Verstellkräfte aufgebracht werden. Somit kann eine aktuelle Auslenkposition sicher gehalten werden, auch wenn beide Zugelemente vorgespannt sind.

Gemäß einer weiteren beispielhaften Ausgestaltung des Instruments bestehen die Zugelemente aus einer superelastischen Legierung. Beispielhaft können die Zugelemente aus Nitinol, etwa aus Nitinol-Drähten gestaltet sein. Superelastische Werkstoffe und Legierungen weisen ein sog. pseudoelastisches bzw. superelastisches Verhalten auf. Allgemein weisen Formgedächtnislegierungen, zu denen auch Nitinol gehört, ein solches Verhalten auf. Ähnlich wie ein idealer Federstahl sind derartige Werkstoffe sehr gut verformbar, wobei nahezu keine plastische Verformung auftritt. Mit anderen Worten können derartige Werkstoffe ihre Ursprungsgestalt auch bei häufigen Lastwechseln und Lastspielen bewahren.

Gemäß einer weiteren beispielhaften Ausgestaltung des Instruments umfasst der Gelenkabschnitt mehrere Gelenkglieder, die gelenkig in Reihe miteinander verbunden sind, wobei die Gelenkglieder mit Anschlägen zur spielfreien Definition zumindest eines Krümmungszustands versehen sind, und wobei die Anschläge benachbarter Gelenkglieder in einem ersten Zustand des Schaftes einander kontaktieren und in einem zweiten Zustand voneinander beabstandet sind.

Dies heißt mit anderen Worten, dass für zumindest eine der beiden Extremlagen, die der Effektor in Bezug auf den Schaft einnehmen kann, eine hochgenaue und wiederholgenaue Positionierung ermöglicht ist. Dies wird durch die Anschläge gewährleistet, die in der jeweiligen Lage einander kontaktieren und eine weitere Bewegung unterbinden.

Beispielhaft ist der Gelenkabschnitt insgesamt aus einer Mittelposition, in der er zur Längsachse des Schaftes ausgerichtet ist, seitlich ausschwenkbar, wobei das seitliche Ausschwenken eine Relativschwenkung zwischen den Gelenkgliedern des Gelenkabschnitts umfasst. Demgemäß können die Gelenkglieder mit Anschlägen versehen sein, die im voneinander beabstandeten Zustand linear und/oder winklig voneinander beabstandet sind.

Gemäß einer weiteren Ausgestaltung des Instruments ist der erste Zustand ein gerader, nicht ausgelenkter Zustand, bei dem sich der Schaft in der Mittelposition befindet, und wobei der zweite Zustand ein ausgelenkter Zustand ist. Gemäß einer beispielhaften Ausgestaltung sind die Gelenkglieder mit weiteren Anschlägen versehen, wobei Anschläge benachbarter Gelenkglieder im zweiten Zustand des Schaftes einander kontaktieren und im ersten Zustand des Schaftes voneinander beabstandet sind.

Die Extremlagen (erster Zustand und zweiter Zustand) können, bezogen auf eine Längsachse des Schafts, eine 0° Stellung und eine 90° Stellung umfassen. Jeder der beiden Extremlagen kann ein Anschlag zugeordnet sein, der durch Einzelanschläge der Gelenkglieder gebildet ist.

Gemäß einer weiteren beispielhaften Ausgestaltung des Instruments ist zur Reibungserhöhung zumindest ein elastischer Klemmkörper vorgesehen ist, insbesondere zumindest ein Elastomerring, der eine Lagesicherung des aktuellen Krümmungszustands des Schaftes bewirkt. Gemäß dieser Ausgestaltung sind beim Auslenkmechanismus bewusste Maßnahmen zur Reibungserhöhung vorgesehen. Vorzugsweise kann die Reibung derart erhöht werden, dass Selbsthemmung auftritt, solange die Handhabungskraft am Steuerhebel ein bestimmtes Maß nicht überschreitet. Insbesondere dann, wenn keine Handhabungskraft auf den Steuerhebel einwirkt, erreicht die Reibung im Auslenkmechanismus vorzugsweise ein Niveau, das den aktuellen Krümmungszustand des Gelenkabschnitts fixiert. In diesem Zusammenhang wird angemerkt, dass zumindest gemäß einigen beispielhaften Ausführungsformen eine inhärente Vorspannung in den Zugelementen gegeben ist. Vorzugsweise ist die Reibung im Auslenkmechanismus derart erhöht, dass diese Basiskräfte zwar das Spiel im System minimieren oder gar eliminieren, jedoch keine Selbstverstellung des Gelenkabschnitts bewirken.

Beispielhaft kann der elastische Klemmkörper beim Steuerhebel vorgesehen sein. Dies kann etwa eine Anordnung umfassen, die zumindest einen O-Ring aus elastischem Material umfasst, der die Schwenkachse umgibt und zwischen dem Steuerhebel und einem Gehäuseteil/Lagerstück des Handhabungsabschnitts unter Vorspannung aufgenommen ist. Dies hat den Vorteil, dass die Lagesicherung wiederum am proximalen Handhabungsabschnitt und nicht am distalen Ende des Schaftes erfolgt. Grundsätzlich ist es auch vorstellbar, derartige Klemmkörper etwa beim Gelenkabschnitt vorzusehen. Dies würde jedoch wiederum den Herstellungsaufwand und Montageaufwand im Bereich des sehr kleinen distalen Endes des Schaftes erhöhen.

Die vorstehend beschriebenen Aspekte und Ausgestaltungen des Instruments erlauben eine verbesserte Funktion und stellen eine Mehrzahl an Freiheitsgraden bereit, die allesamt spielarm oder spielfrei gestaltet sind. Gleichwohl ist das Instrument trotzdem miniaturisierbar. Das Gewicht des Instruments kann sehr niedrig ausfallen. Es ergibt sich beim Instrument ein günstiger Schwerpunkt für den Operateur. Ferner ergibt sich ein günstiges Sichtfeld, da das Instrument sehr kompakt und integral gestaltet ist.

Weitere Merkmale und Vorteile der Erfindung ergeben sich aus der nachfolgenden Beschreibung bevorzugter Ausführungsbeispiele unter Bezugnahme auf die Zeichnungen. Es zeigen:
- Fig. 1: eine perspektivische Ansicht eines neurochirurgischen Instruments, vom distalen Ende her;
- Fig. 2: eine gebrochene Seitenansicht des Instruments gemäß Fig. 1 in einem ersten Schwenkzustand;
- Fig. 3: eine vergrößerte Ansicht des distalen Endes des Instruments gemäß Fig. 2;
- Fig. 4: eine gebrochene Seitenansicht des Instruments gemäß Fig. 1 in einem zweiten Schwenkzustand, der vom Zustand gemäß Fig. 2 abweicht;
- Fig. 5: eine vergrößerte Ansicht des distalen Endes des Instruments gemäß Fig. 4;
- Fig. 6: eine seitliche Teilansicht eines Auslenkmechanismus eines neurochirurgischen Instruments;
- Fig. 7: eine geschnittene Ansicht der Anordnung gemäß Fig. 6;
- Fig. 8: eine geschnittene vergrößerte Ansicht eines Gelenkabschnitts am Schaft eines Instruments, wobei der Auslenkzustand des Gelenkabschnitts mit dem in den Figuren 6 und 7 gezeigten Zustand des Auslenkmechanismus übereinstimmt;
- Fig. 9: eine Anordnung gemäß Fig. 6 in einem zweiten Schwenkzustand;
- Fig. 10: eine Anordnung gemäß Fig. 7 in einem zweiten Schwenkzustand;
- Fig. 11: eine Anordnung gemäß Fig. 8 in einem zweiten Schwenkzustand;
- Fig. 12: eine perspektivische explodierte Teilansicht einer Steuereinheit für einen Auslenkmechanismus eines Instruments;
- Fig. 13: eine weitere perspektivische explodierte Darstellung der Steuereinheit gemäß Fig. 12 in einer von Fig. 12 abweichenden Orientierung;
- Fig. 14: eine vergrößerte Schnittansicht einer Steuereinheit eines Auslenkmechanismus für ein Instrument in einem Zustand, der dem in den Figuren 9, 10 und 11 gezeigten Zustand ähnelt;
- Fig. 15: eine isolierte Darstellung eines Steuerhebels für die in Fig. 14 gezeigte Steuereinheit;
- Fig. 16: eine Seitenansicht eines distalen Endes eines neurochirurgischen Instruments, das mit einem geschlossenen Effektor versehen ist;
- Fig. 17: eine perspektivische Ansicht der Anordnung gemäß Fig. 16;
- Fig. 18: eine explodierte Darstellung der Anordnung gemäß den Figuren 16 und 17 in einer Orientierung gemäß Fig. 17;
- Fig. 19: eine Darstellung der Anordnung gemäß Fig. 16 in einem geöffneten Zustand des Effektors;
- Fig. 20: eine weitere Darstellung der Anordnung gemäß Fig. 17 in einem geöffneten Zustand des Effektors;
- Fig. 21: eine weitere Darstellung der Anordnung gemäß Fig. 18 in einem geöffneten Zustand des Effektors;
- Fig. 22: eine rückwärtige perspektivische Ansicht eines Koppelmechanismus für ein neurochirurgisches Instrument, zur Steuerung eines Effektors;
- Fig. 23: eine vergrößerte Anordnung des Koppelmechanismus gemäß Fig. 22, wobei aus Veranschaulichungszwecken weitere Komponenten ausgeblendet sind;
- Fig. 24: eine perspektivische rückwärtige Ansicht einer Schnittstelle für ein neurochirurgisches Instrument;
- Fig. 25: eine weitere rückwärtige perspektivische Ansicht einer Schnittstelle für ein neurochirurgisches Instrument, in einer von Fig. 24 abweichenden Orientierung, wobei in Fig. 25 aus Veranschaulichungsgründen ein Verschlussbügel isoliert dargestellt ist;
- Fig. 26: eine vergrößerte geschnittene Darstellung eines an einer Schnittstelle gefügten neurochirurgischen Instruments; und
- Fig. 27: eine weitere seitliche geschnittene Darstellung eines Instruments, etwa gemäß Fig. 26, wobei in Fig. 27 eine Schaftbaugruppe und ein Griffstück des Instruments voneinander gelöst sind.

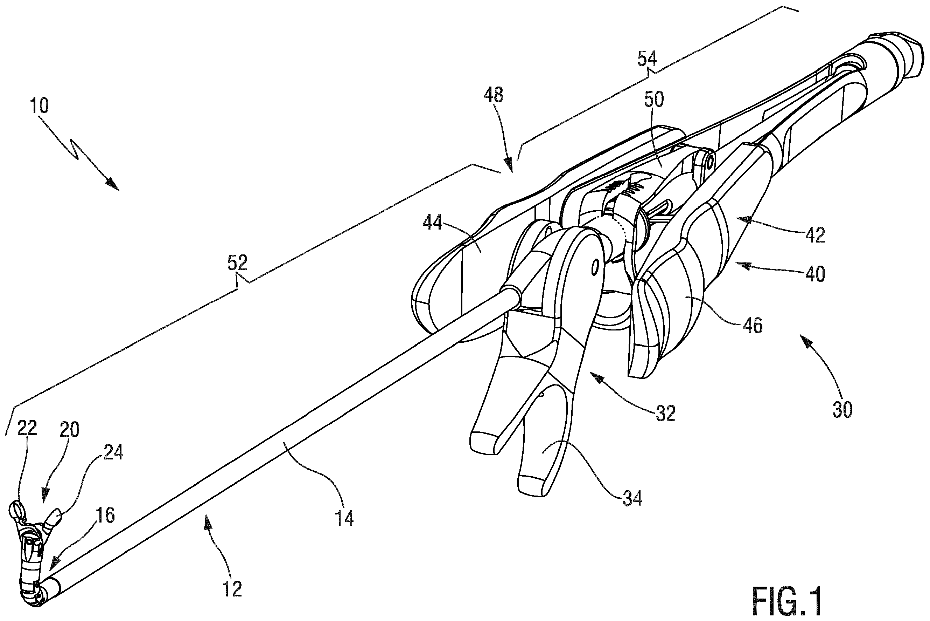

Fig. 1 zeigt anhand einer perspektivischen Darstellung ein Instrument 10, dessen distales Ende dem Betrachter zugewandt und dessen proximales Ende vom Betrachter abgewandt ist. Das Instrument 10 ist als Instrument für die Neurochirurgie und/oder die Hirnchirurgie gestaltet. Demgemäß kann das Instrument 10 auch als neurochirurgisches Instrument bezeichnet werden.

Das Instrument 10 weist einen Schaft 12 auf, der zumindest abschnittsweise durch ein Rohr 14 gebildet ist, das starr gestaltet ist. Das Rohr 14 definiert eine Längsachse des Schaftes 12, die sich von einem proximalen Ende hin zu einem distalen Ende erstreckt. Im Bereich des distalen Endes ist dem Schaft 12 ein Gelenkabschnitt 16 zugeordnet, der in Fig. 1 beispielhaft in einer ausgelenkten und/oder abgewinkelten Stellung gezeigt ist. Ferner ist am distalen Ende des Schaftes 12 ein Effektor 20 aufgenommen, der etwa als Greifer gestaltet ist. Der Effektor 20 umfasst ein erstes Maulteil 22 und ein zweites Maulteil 24. In Fig. 1 ist der Effektor 20 in einer geöffneten Stellung gezeigt, in der die Maulteile 22, 24 geöffnet sind.

An das proximale Ende des Schaftes 12 schließt sich ein Handhabungsabschnitt 30 an, in dem das Instrument 10 durch einen Benutzer, etwa einen Chirurgen oder Operateur, aufgenommen und geführt werden kann. Der Schaft 12 definiert einen distalen Abschnitt des Instruments. Der Handhabungsabschnitt 30 definiert einen proximalen Abschnitt des Instruments 10.

Das Instrument 10 umfasst ferner einen Auslenkmechanismus 32 zum Steuern der Auslenkung und/oder Winkelposition des Gelenkabschnitts 16. Der Auslenkmechanismus 32 ist beispielhaft mit einem Steuerhebel 34 versehen. Ferner weist das Instrument 10 einen Betätigungsmechanismus 40 für den Effektor 20 auf. Der Betätigungsmechanismus 40 umfasst eine Handhabe 42, die einen ersten Arm 44 und einen zweiten Arm 46 aufweist. Die Arme 44, 46 erstrecken sich ausgehend von einem proximalen Ende des Handhabungsabschnitts 30 nach distal in Richtung auf den Schaft 12.

Ferner weist das Instrument 10 eine Schnittstelle 48 auf, der ein Verschlussbügel 50 zugeordnet ist. An der Schnittstelle 48 kann das Instrument 10 in eine Schaftbaugruppe 52 und ein Griffstück 54 zerlegt werden. Die Schaftbaugruppe 52 ist eine distale Baugruppe. Das Griffstück 54 bezeichnet eine proximale Baugruppe. Der Schaftbaugruppe 52 ist zumindest abschnittsweise der Schaft 12 zugeordnet. Dem Griffstück 54 ist zumindest abschnittsweise der Handhabungsabschnitt 30 zugeordnet. Gemäß dem anhand der Fig. 1 gezeigten Ausführungsbeispiel ist zumindest der Auslenkmechanismus 32, insbesondere der Steuerhebel 34 der Schaftbaugruppe 52 zugeordnet, also distalseitig von der Schnittstelle 48 angeordnet. Andere Gestaltungen und Zuordnungen betreffen den Auslenkmechanismus 32 und/oder den Betätigungsmechanismus 40 sind denkbar.

Der Verschlussbügel 50 ist dazu ausgestaltet, die Schaftbaugruppe 52 und das Griffstück 54 miteinander zu verriegeln. Der Verschlussbügel 50 ist verschwenkbar, um ein Lösen der Schaftbaugruppe 52 vom Griffstück 54 zu erlauben.

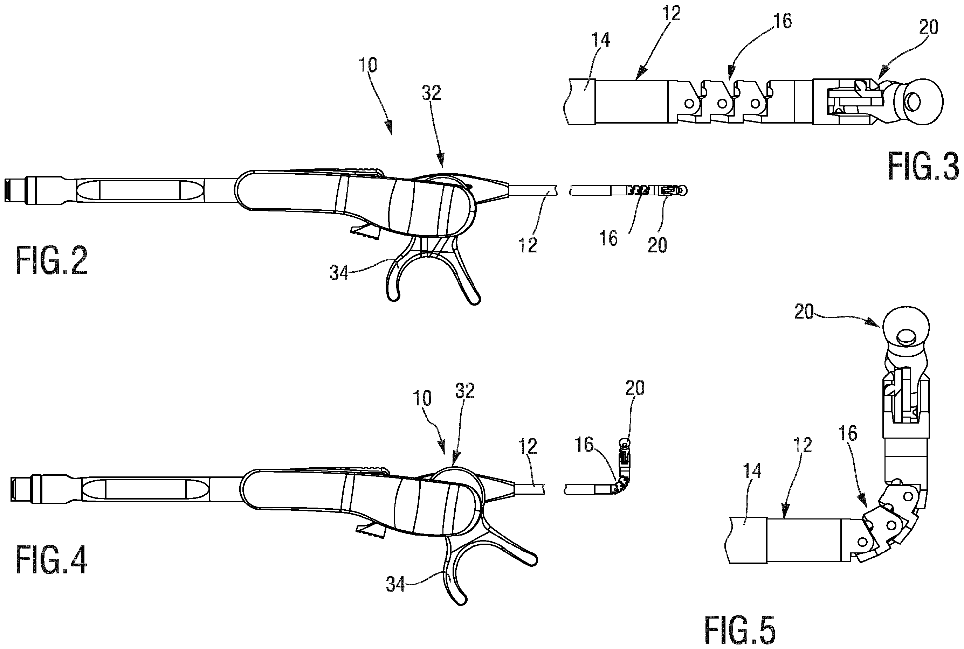

Ergänzend wird auf die Figuren 2, 3, 4 und 5 verwiesen. Fig. 1, Fig. 2 und Fig. 4 zeigen, dass die Handhabe 42 des Betätigungsmechanismus 40 an einem distalen Ende des Griffstücks 54 aufgenommen ist, wobei die Arme 44, 46 auslenkbar sind. Die Arme 44, 46 erstrecken sich zumindest abschnittsweise seitlich vom Verschlussbügel 50 und vom Steuerhebel 34 des Auslenkmechanismus 32. Mit anderen Worten sind der Verschlussbügel 50 und der Steuerhebel 34 zumindest abschnittsweise zwischen den Armen 44, 46 der Handhabe 42 angeordnet. Dies sorgt insgesamt für eine gute Ergonomie und für ein freies Sichtfeld für den Operateur. Ferner erlaubt die in Fig. 1 gezeigte Konfiguration eine gute Handhabbarkeit. Das Instrument 10 weist darüber hinaus einen günstigen Schwerpunkt auf, der das Halten und Führen des Instruments 10 vereinfacht.

Die Figuren 2 und 4 zeigen gebrochene Seitenansichten des in Fig. 1 perspektivisch dargestellten Instruments 10. Fig. 2 zeigt einen ersten Schwenkzustand. Fig. 4 zeigt einen zweiten Schwenkzustand. Fig. 3 zeigt eine vergrößerte Darstellung eines distalen Bereichs des Schaftes 12 des Instruments 10, wobei der Schwenkzustand des Gelenkabschnitts 16 dem in Fig. 2 gezeigten Zustand entspricht. Fig. 5 zeigt eine vergrößerte Ansicht des distalen Endes des Schaftes, wobei der in Fig. 5 gezeigte Zustand des Gelenkabschnitts 16 dem in Fig. 4 gezeigten Schwenkzustand entspricht.

Aus den Figuren 2 und 4 wird ersichtlich, dass der Steuerhebel 34 des Auslenkmechanismus 32 verschwenkbar ist, um den Gelenkabschnitt 16 zwischen einem ersten Krümmungszustand (Fig. 2) und einem zweiten Krümmungszustand (Fig. 4) zu bewegen. Fig. 2 zeigt einen Zustand, in dem der Gelenkabschnitt 16 mittig ausgerichtet und konzentrisch zur Längsachse des Schaftes 12 orientiert ist. Fig. 5 zeigt einen Zustand, in dem der Gelenkabschnitt 16 maximal ausgelenkt bzw. abgewinkelt ist.

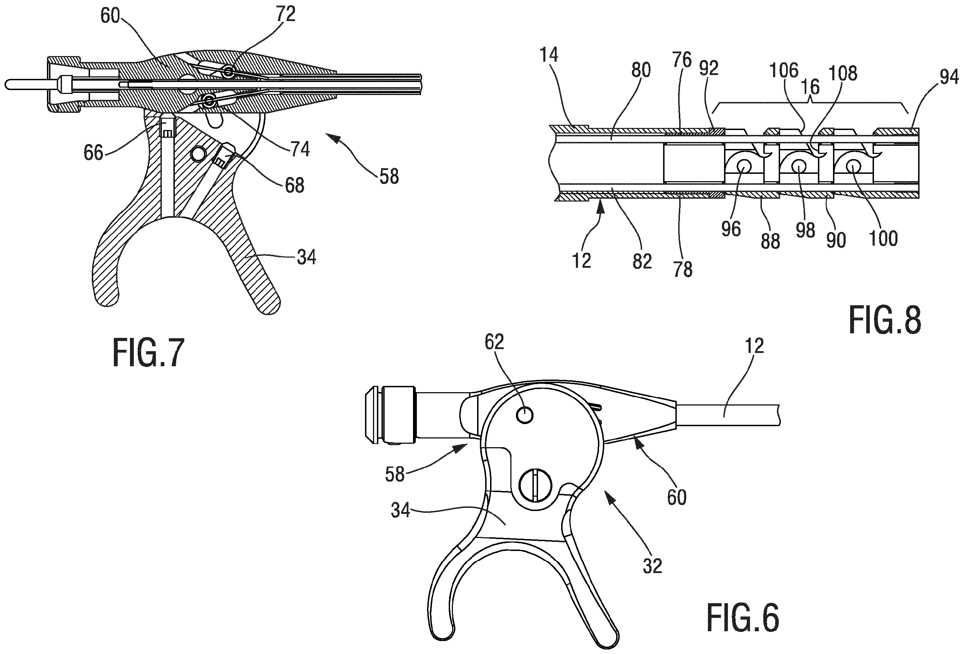

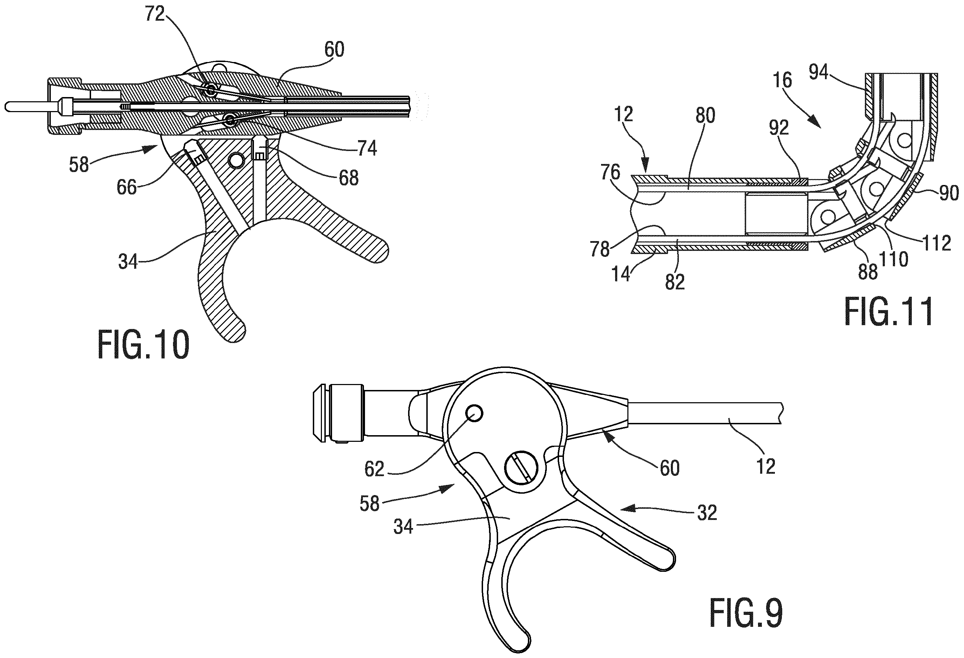

Der in Fig. 2 und Fig. 3 gezeigte erste Schwenkzustand wird anhand der Figuren 6, 7 und 8 näher veranschaulicht. Der in den Figuren 4 und 5 gezeigte zweite Krümmungszustand wird anhand der Figuren 9, 10 und 11 näher veranschaulicht.

Fig. 6 und Fig. 9 veranschaulichen eine Verschwenkbarkeit des Steuerhebels 34. Fig. 7 und Fig. 10 zeigen korrespondierende geschnittene Ansichten. Fig. 8 zeigt einen Schnitt durch den Gelenkabschnitt 16 im ersten Krümmungszustand. Fig. 11 zeigt einen Schnitt durch den Gelenkabschnitt 16 im zweiten Krümmungszustand.

Fig. 6 und Fig. 7 zeigen, dass der Auslenkmechanismus 32 ferner eine Steuereinheit 58 umfasst, der der Steuerhebel 34 zugeordnet ist. Der Steuerhebel 34 ist verschwenkbar an einem Lagerstück 60 aufgenommen. Eine Schwenkachse 62 für den Steuerhebel 34 ist in Fig. 6 und Fig. 9 mit 62 bezeichnet. Bei der Bewegung zwischen den in Fig. 6 und Fig. 9 gezeigten Zuständen wird der Steuerhebel 34 um die Schwenkachse 62 verschwenkt.

Fig. 7 und Fig. 10 ist ergänzend entnehmbar, dass der Steuerhebel 34 gemäß beispielhaften Ausgestaltungen mit Einstellschrauben 66, 68 versehen ist, die eine Feineinstellung und/oder Justierung von Winkelpositionen des Steuerhebels 34 in Bezug auf das Lagerstück 60 ermöglichen. Fig. 7 zeigt die Steuereinheit 58 in einem Zustand, in dem sich der Gelenkabschnitt 16 im ersten Krümmungszustand befindet, vgl. auch Fig. 8. In diesem Zustand kann die Einstellschraube 66 das Lagerstück 60 kontaktieren und dort einen einstellbaren Anschlag für den Steuerhebel 34 bereitstellen.

Fig. 10 veranschaulicht hingegen einen zweiten Zustand des Steuerhebels 34, der dem in Fig. 11 gezeigten zweiten Krümmungszustand des Gelenkabschnitts 16 entspricht. Im zweiten Zustand des Steuerhebels 34 kontaktiert die Einstellschraube 68 das Lagerstück 60. Demgemäß steht auch im zweiten Zustand ein einstellbarer Anschlag zur Verfügung, über den eine Feineinstellung und/oder Justierung des Schwenkzustands des Steuerhebels 34 in Bezug auf das Lagerstück 60 erfolgen kann.

Die Figuren 7 und 10 zeigen ferner, dass der Steuerhebel 34 und das Lagerstück 60 über Gleitstücke 72, 74 miteinander gekoppelt sind. Ein erstes Gleitstück 72 ist einem ersten Zugelement 76 zugeordnet. Ein zweites Gleitstück 74 ist einem zweiten Zugelement 78 zugeordnet, vgl. auch Fig. 8 und Fig. 11.

Das Gleitstück 72 ist an einem proximalen Ende des Zugelements 76 zur Bewegungsmitnahme mit dem Zugelement 76 verbunden. Das Gleitstück 74 ist an einem proximalen Ende des Zugelements 78 zur Bewegungsmitnahme mit dem Zugelement 78 verbunden.

Das Zugelement 76 ist beispielhaft als Zugdraht 80 ausgeführt. Das Zugelement 78 ist beispielhaft als Zugdraht 82 ausgeführt. Die Zugelemente 76, 78 sind vorzugsweise aus einem hochelastischen Werkstoff gefertigt. Hierbei kann es sich bspw. um Nitinol und/oder eine ähnliche superelastische Legierung handeln.

Insbesondere die gekrümmte Darstellung in Fig. 11 zeigt, dass das erste Zugelement 76 auch als inneres Zugelement bezeichnet werden kann. Das Zugelement 78 kann auch als äußeres Zugelement bezeichnet werden, bezogen auf den jeweiligen Krümmungsradius der Zugelemente 76, 78. Demgemäß kann der Zugdraht 80 auch als innerer Zugdraht bezeichnet werden. Der Zugdraht 82 kann auch als äußerer Zugdraht bezeichnet werden. Durch einen Zug am Zugelement 76 nach distal kann der Gelenkabschnitt 16 ausgelenkt und/oder abgewinkelt werden, vgl. Fig. 11. Durch einen Zug am Zugelement 78 kann der Gelenkabschnitt 16 eingeklappt oder zurückgefahren werden, um seine Ausgangsposition einzunehmen, die etwa einer mittigen Stellung entspricht, vgl. auch Fig. 8.

Somit bewirkt ein Verschwenken des Steuerhebels 34 in Richtung auf das distale Ende des Instruments 10 ein Auslenken des Gelenkabschnitts 16 in eine abgewinkelte Stellung. Umgedreht erzeugt eine Schwenkbewegung des Steuerhebels 34 in Richtung auf das proximale Ende des Instruments 10 einen Zug am Zugelement 78. Auf diese Weise wird der Gelenkabschnitt 16 aus der gekrümmten wieder in die geradlinige, mittige Position bewegt.

Der Gelenkabschnitt 16 wird anhand der Fig. 8 und der Fig. 11 näher veranschaulicht. Der Gelenkabschnitt 16 umfasst beispielhaft ein Gelenkglied 88 und ein Gelenkglied 90, die zwischen einem proximalen Verbindungsstück 92 und einem distalen Verbindungsstück 94 aufgenommen sind. Schwenkachsen 96, 98, 100 veranschaulichen, dass die Anordnung umfassend das Verbindungsstück 92, das Gelenkglied 88, das Gelenkglied 90 und das Verbindungsstück 94 insgesamt verschwenkbar ist, umfassend jeweils eine Relativverschwenkung benachbarter Glieder.

Die Gelenkglieder 88, 90 und vorzugsweise auch die Verbindungsstücke 92, 94 sind vorzugsweise mit Anschlägen 106, 108, 110, 112 versehen. In Fig. 8 sind beispielhaft einander gegenüberliegende Anschläge 106, 108 dargestellt, wobei der Anschlag 106 dem Gelenkglied 88 und der Anschlag 108 dem Gelenkglied 90 zugeordnet ist. Es versteht sich, dass weitere Anschläge auf dieser Seite vorhanden sind. Fig. 11 zeigt, dass im gekrümmten Zustand des Gelenkabschnitts 16 die Anschläge 106, 108 einander kontaktieren.

In ähnlicher Weise zeigt Fig. 11, dass ferner Anschläge 110, 112 vorgesehen sind, die eine geradlinige, gestreckte Orientierung des Gelenkabschnitts 16 definieren. Der Anschlag 110 ist dem Gelenkglied 88 zugeordnet. Der Anschlag 112 ist dem Gelenkglied 90 zugeordnet. Weitere beteiligte Glieder sind in ähnlicher Weise mit Anschlägen versehen. Ein Vergleich zwischen Fig. 8 und Fig. 11 zeigt, dass die Anschläge 110, 112 genutzt werden, um die geradlinige, mittige Orientierung des Gelenkabschnitts 16 definieren. Zumindest die mittige Stellung des Gelenkabschnitts 16 wird durch die Anschläge 110, 112 hochgenau und wiederholbar definiert.

In ähnlicher Weise können die Anschläge 106, 108 dazu ausgestaltet sein, auch für den abgewinkelten/ausgelenkten Zustand des Gelenkabschnitts 16 eine hohe Positioniergenauigkeit und Wiederholbarkeit zu gewährleisten.

Die in Fig. 8 und Fig. 11 gezeigte Ausführungsform zeigt ferner, dass die Zugelemente 76, 78 zwar genutzt werden, um den Gelenkabschnitt 16 zu verschwenken und/oder zurückzuführen. Die eigentliche Positionierung in den jeweiligen Endlagen erfolgt jedoch durch die Anschläge 106, 108 für die in Fig. 11 gezeigte Stellung und die Anschläge 110, 112 für die in Fig. 8 gezeigte Stellung.

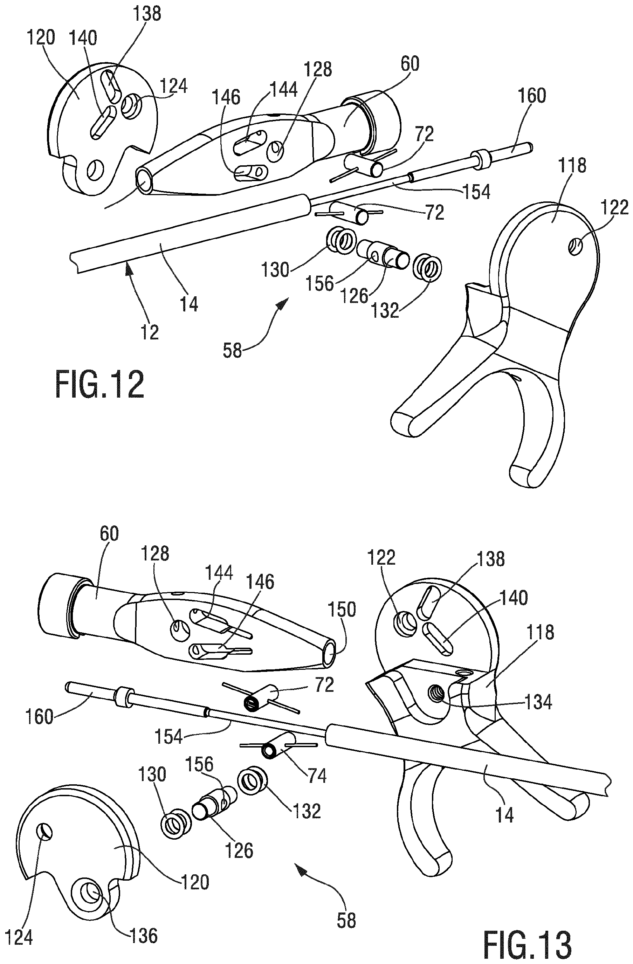

Mit Bezugnahme auf Fig. 12 und Fig. 13 wird eine beispielhafte Ausgestaltung der Steuereinheit 58 für den Auslenkmechanismus 32 weiter veranschaulicht. Fig. 12 und Fig. 13 zeigen jeweils explodierte perspektivische Ansichten, wobei den Ansichten jeweils unterschiedliche Orientierungen zugrunde gelegt sind.

Beispielhaft ist der Steuerhebel 34 zweiteilig ausgeführt und mit einem ersten Seitenteil 118 und einem zweiten Seitenteil 120 versehen. Die Seitenteile 118, 120 können etwa miteinander verschraubt werden. Das erste Seitenteil 118 ist mit einer Lagerausnehmung 122 versehen. Das zweite Seitenteil 120 ist mit einer Lagerausnehmung 124 versehen.

Zur schwenkbaren Aufnahme des Steuerhebels 34 ist ein Lagerbolzen 126 vorgesehen, der in einer Lagerausnehmung 128 am Lagerstück 60 aufnehmbar ist. Das Seitenteil 118 ist über die Lagerausnehmung 122 am Lagerbolzen 126 aufgenommen. Das Seitenteil 120 ist über die Lagerausnehmung 124 am Lagerbolzen 126 aufgenommen. Demgemäß kann der Steuerhebel 34, der die Seitenteile 118, 120 umfasst, um den Lagerbolzen 126 verschwenkt werden.

Es versteht sich, dass der Lagerbolzen 126 auch integral am Lagerstück 60 ausgebildet sein kann. Gemäß dieser beispielhaften Ausgestaltung ist keine separate Lagerausnehmung 128 am Lagerstück 60 vorgesehen.

In den Figuren 12 und 13 sind ferner mit 130, 132 elastische Klemmkörper angedeutet, die zur Reibungserhöhung bzw. Positionssicherung vorgesehen sind. Die Klemmkörper 130, 132 können beispielhaft als sog. O-Ringe ausgeführt sein. Die Figuren 12 und 13 zeigen, dass jedem seitlichen Ende des Lagerbolzens 126 zwei Klemmkörper 130, 132 zugeordnet sind. Der oder die Klemmkörper 130 ist/sind zwischen dem Lagerbolzen 126 und der Lagerausnehmung 122 vorgesehen. Der oder die Klemmkörper 132 ist/sind zwischen dem Lagerbolzen 126 und der Lagerausnehmung 124 vorgesehen. Die Seitenteile 118, 120 des Steuerhebels 34 weisen ferner Montageöffnungen 134, 136 auf. Die am Seitenteil 118 vorgesehene Montageöffnung 134 umfasst beispielhaft ein Gewinde. Die am Seitenteil 120 vorgesehene Montageöffnung 136 umfasst beispielhaft ein Durchgangsloch für eine Schraube.

Im gefügten Zustand ist der Steuerhebel 34 um den Lagerbolzen 126 verschwenkbar, wobei die Klemmkörper 130, 132 eine Reibungserhöhung bewirken, die sich in einer Selbsthemmung oder Positionssicherung niederschlagen kann. Mit anderen Worten ist es bevorzugt, wenn der Steuerhebel 34 selbsttätig und ohne äußere Einwirkung seine Ist-Schwenkposition relativ zum Lagerstück 60 beibehält. Die entsprechenden Reibkräfte werden vorrangig durch die Klemmkörper 130, 132 erzeugt, die mit Vorspannung zwischen dem Lagerstück 60 und dem Seitenteil 118 sowie zwischen dem Lagerstück 60 und dem Seitenteil 120 aufgenommen sind.

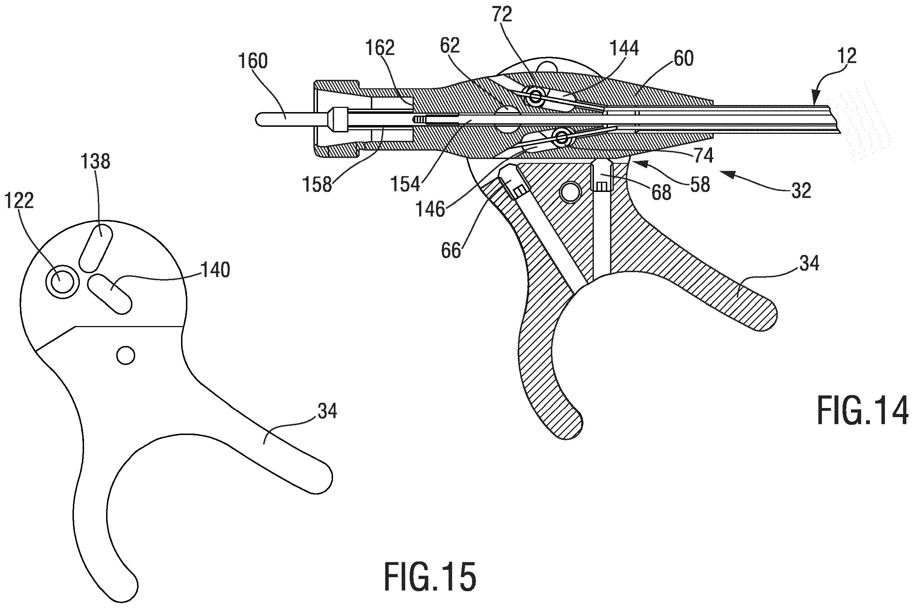

Ergänzend wird zu den Figuren 12 und 13 auf Fig. 14 und Fig. 15 verwiesen. Fig. 14 zeigt eine geschnittene Ansicht der Steuereinheit 58 für den Auslenkmechanismus 32. Fig. 15 zeigt eine isolierte Seitendarstellung eines Steuerhebels 34.

Am Steuerhebel 34 ist eine erste Führungsbahn 138 und eine zweite Führungsbahn 140 ausgebildet. Ferner zeigen insbesondere Fig. 12 und Fig. 13, dass am Lagerstück 60 eine erste Führungsbahn 144 und zweite Führungsbahn 146 ausgebildet ist.

Das Gleitstück 72 für das erste Zugelement 76 ist im gefügten Zustand in der Führungsbahn 144 des Lagerstücks 60 und der Führungsbahn 138 des Steuerhebels 34 aufgenommen. Das Gleitstück 74, das dem Zugelement 78 zugeordnet ist, ist in der Führungsbahn 146 des Lagerstücks 60 und der Führungsbahn 140 des Steuerhebels 34 aufgenommen.

Bei der Schwenkbewegung des Steuerhebels 34 um die Schwenkachse 62 (vgl. auch Fig. 6 und Fig. 9) erfolgt über die Führungsbahnen 138, 140 ein Antrieb der Gleitstücke 72, 74, vgl. auch Fig. 15. Die Gleitstücke 72, 74 sind mit den Zugelementen 76, 78 gekoppelt. Auf diese Weise kann der Gelenkabschnitt 16 am distalen Ende des Instruments gestreckt oder abgewinkelt werden.

Fig. 15 zeigt, dass die Führungsbahnen 138, 140 am Steuerhebel 34 in bestimmter Weise relativ zur Lagerausnehmung 122, 124 aufgenommen sind. Dies bewirkt, dass beim Verschwenken des Steuerhebels 34 um die Schwenkachse 62 die Zugelemente 76, 78 nicht um das gleiche Maß verschoben werden. Diese Maßnahme hat den Vorteil, dass unterschiedliche Biegeradien der Zugelemente 76, 78 kompensiert werden können, vgl. hierzu auch Fig. 11.

Ein weiteres Ziel der anhand der Figuren 12 bis 15 veranschaulichten Ausgestaltung der Steuereinheit 58 ist, dass nach Möglichkeit beide Zugelemente 76, 78 gleichzeitig unter Zugspannung stehen, wenn der Steuerhebel 34 nach proximal oder nach distal verschwenkt wird. Wie bereits vorstehend in Zusammenhang mit Fig. 8 und Fig. 11 erläutert, reicht es grundsätzlich aus, zum Auslenken/Abwinkeln das erste Zugelement 76 zu ziehen. Umgedreht reicht es aus, das zweite Zugelement 78 nach distal zu bewegen, um den Gelenkabschnitt 16 zurückzuführen in eine gestreckte, mittige Stellung.

Gemäß beispielhaften Aspekten der vorliegenden Ausgestaltung ist die Steuereinheit 58 derart gestaltet, dass jeweils beide Zugelemente 76, 78 in ihrer Längsrichtung zumindest leicht unter Vorspannung stehen. Dies hat den Vorteil, dass der Auslenkmechanismus 32 spielarm oder nahezu spielfrei gestaltet ist. Insbesondere bei einer Bewegungsumkehr kann somit ein übermäßiges Spiel vermieden werden.

Im Lagerstück 60 erstreckt sich ein Durchgang 150 zwischen einem distalen und einem proximalen Ende. Der Durchgang 150 ist beispielhaft konzentrisch zu einer Längsachse durch den Schaft 12. Fig. 14 zeigt einen Zustand des Instruments 10, in dem der Schaft 12 und das Lagerstück 60 miteinander gefügt sind, wobei im Schaft 12 ein Schubstück 154 angeordnet ist, das sich durch den Durchlass 156 im Lagerbolzen 126 erstreckt.

Fig. 12, Fig. 13 und Fig. 14 zeigen ferner, dass neben den Zugelementen 76, 78 auch das Schubstück 154 am Schaft 12, insbesondere im Rohr 14, aufgenommen ist. Am Lagerbolzen 126 ist ein Durchlass 156 für das Schubstück 154 vorgesehen, so dass dieses zentral durch den Lagerbolzen 126 hindurchgeführt werden kann.

Am proximalen Ende des Schubstücks 154 ist ein Druckstück 160 vorgesehen, das mit dem Schubstück 154 verbunden ist. In Fig. 14 ist ferner mit 158 eine Druckfeder oder Feder angedeutet, die zwischen dem Druckstück 160 und einem Anschlag 162 am Lagerstück 60 aufgenommen ist, um das Schubstück 154 in Richtung auf das proximale Ende zu drängen. Auch dies kann zur spielarmen oder sogar spielfreien Gestaltung der Steuereinheit 58 und des Auslenkmechanismus 32 beitragen.

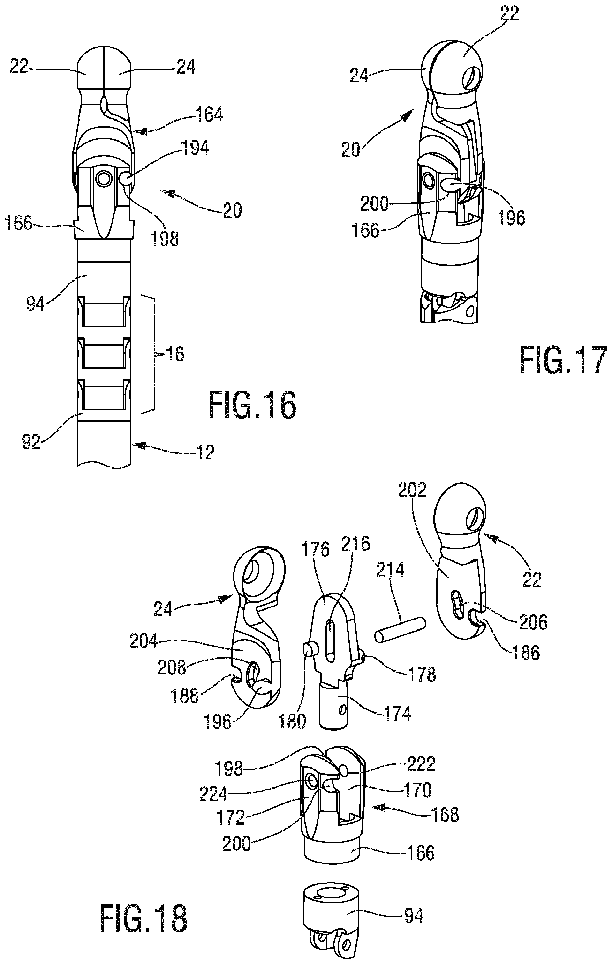

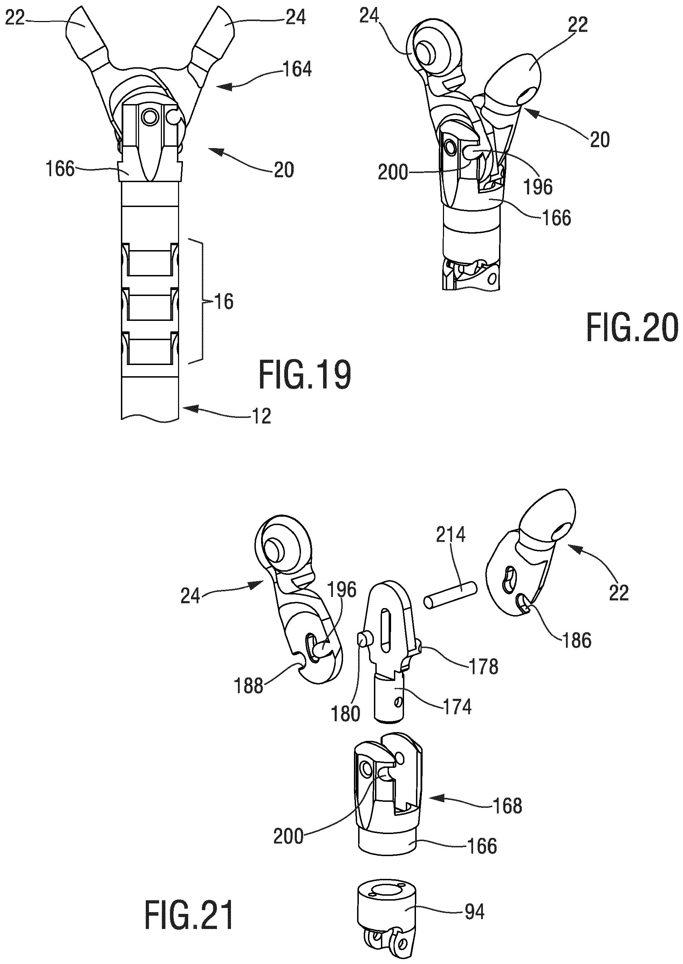

Ergänzend wird auf die Figuren 16, 17 und 18 sowie auf die Figuren 19, 20 und 21 verwiesen. Fig. 16, Fig. 17 und Fig. 18 zeigen einen am distalen Ende des Schaftes aufgenommenen Effektor 20, der beispielhaft als Greifer 164 gestaltet ist. Es versteht sich, dass der Effektor 20 alternativ auch als Zange, Kneifer, Schere, Klemme und in ähnlicher Weise ausgebildet sein kann.

Fig. 19, Fig. 20 und Fig. 21 zeigen den Effektor 20 in einer geöffneten Stellung. Fig. 16, Fig. 17 und Fig. 18 zeigen den Effektor 20 in einer geschlossenen Stellung. Fig. 19 korrespondiert mit Fig. 16. Fig. 20 korrespondiert mit Fig. 17. Fig. 21 korrespondiert mit Fig. 18.

Am Verbindungsstück 94, das mit zumindest einem Gelenkglied 88, 90 (vgl. Fig. 8 und Fig. 11) gekoppelt ist, ist ein Kopfstück 166 aufgenommen, das beispielhaft als Gabelkopf 168, ausgeführt ist. Das Kopfstück 166 umfasst ein erstes Seitenteil 170 und ein zweites Seitenteil 172. Zwischen den Seitenteilen 170, 172 ist ein Koppelstück 174 verschiebbar aufgenommen, wobei das Koppelstück 174 ein Flachstück 176 an seinem distalen Ende umfasst. Vom Flachstück 176 aus erstreckt sich ein Mitnehmer 178 sowie ein weiterer Mitnehmer 180. Die Mitnehmer 178, 180 sind an voneinander abgewandten Seiten des Flachstücks 176 aufgenommen.

Über die Mitnehmer 178, 180 ist das Koppelstück 174 mit dem ersten Maulteil 22 und dem zweiten Maulteil 24 gekoppelt. Zu diesem Zweck ist am ersten Maulteil 22 eine Mitnahmeausnehmung 186 vorgesehen. Ferner ist am zweiten Maulteil 24 eine zweite Mitnahmeausnehmung 188 vorgesehen. Im montierten Zustand des Instruments 10 greift der Mitnehmer 178 in die Mitnahmeausnehmung 186 am Maulteil 22 ein. Demgemäß greift der Mitnehmer 180 in die Mitnahmeausnehmung 188 am zweiten Maulteil 24 ein.

Ferner weist das erste Maulteil 22 einen Vorsprung 194 auf. Daneben weist das zweite Maulteil 24 einen Vorsprung 196 auf. Dem ersten Vorsprung 194 ist eine Lagerausnehmung 198 am Kopfstück 166 zugeordnet. Dem zweiten Vorsprung 196 ist eine Lagerausnehmung 200 am Kopfstück 166 zugeordnet. Der Vorsprung 194 erstreckt sich von einem Flachstück 202 des ersten Maulteil 22. Der Vorsprung 196 erstreckt sich von einem Flachstück 204 des zweiten Maulteils 24.

Im Flachstück 202 ist eine Führungsbahn 206 ausgebildet. Im Flachstück 204 ist eine Führungsbahn 208 ausgebildet. Im gefügten Zustand des Effektors 20 und eines Betätigungsmechanismus 40 für den Effektor 20 ist ferner ein Führungsstift 214 vorgesehen, der eine Führungsausnehmung 216 im Flachstück 176 des Kopfstücks 166 durchragt. Der Führungsstift 214 ist an einem Sitz 222 am Seitenteil 170 und einem Sitz 224 am Seitenteil 172 aufgenommen. Zwischen den Seitenteilen 170, 172 sind das Koppelstück 174 sowie das erste Maulteil 22 und das zweite Maulteil 24 aufgenommen, insbesondere über ihre Flachstücke 176, 202, 204. Das Flachstück 176 des Koppelstücks 174 ist zwischen den Flachstücken 202, 204 der Maulteile 22, 24 aufgenommen. Der sich zwischen den Sitzen 222, 224 erstreckende Führungsstift 214 durchragt ferner die Führungsbahn 206 am ersten Maulteil 22, die Führungsbahn 208 am Maulteil 24 sowie die Führungsausnehmung 216 im Flachstück 176 des Koppelstücks 174.

Ein Vergleich von Fig. 16, Fig. 17 und Fig. 18 mit Fig. 19, Fig. 20 und Fig. 21 zeigt, dass das Koppelstück 174 nach distal geschoben wird, um die Maulteile 22, 24 zu schließen. Sofern das Koppelstück 174 nach proximal gedrückt oder geschoben wird, öffnen sich die Maulteile 22, 24.

Das bereits in Zusammenhang mit den Figuren 12, 13 und 14 beschriebene Schubstück 154 ist mit dem Koppelstück 174 verbunden, um dieses zum Schließen oder Öffnen des Effektors 20 zu verfahren. Grundsätzlich kann auch das Schubstück 154 aus einem hochelastischen Material oder sogar aus einer superelastischen Legierung, wie etwa Nitinol, bestehen.

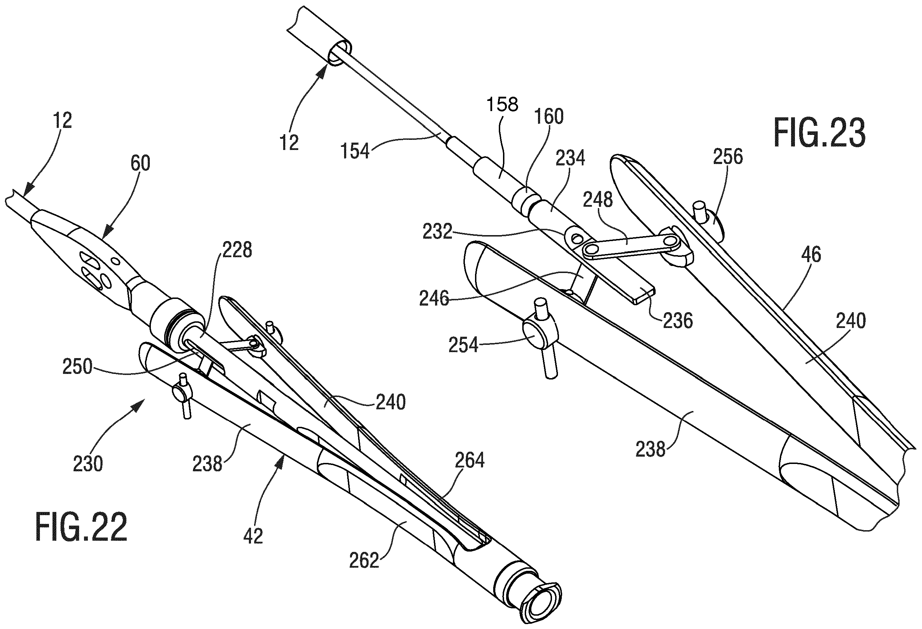

Mit ergänzender Bezugnahme auf Fig. 22 und Fig. 23 wird der Betätigungsmechanismus 40 für den Effektor 20 näher veranschaulicht. Wie bereits in Zusammenhang mit Fig. 1 erläutert wurde, ist die Handhabe 42 des Betätigungsmechanismus 40 am distalen Ende des Instruments 10 aufgenommen. Die Handhabe 42 ist an einem Trägerschaft 228 aufgenommen, der mit dem Lagerstück 60 und mit dem Schaft 12 in Reihe verbunden ist. In Fig. 23 sind aus Veranschaulichungsgründen das Lagerstück 60 und der Trägerschaft 228 ausgeblendet. Ferner sind die Arme 44, 46 der Handhabe 42 in den Figuren 22 und 23 nicht explizit dargestellt (vgl. hierzu Fig. 1). Die Arme 44, 46 sind als Aufsatzteile gestaltet.

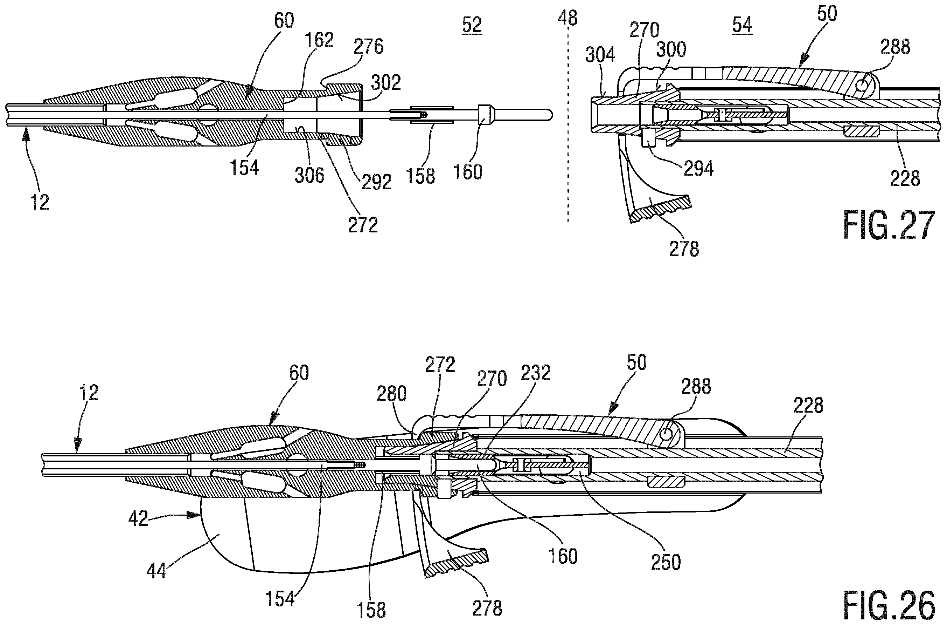

Der Betätigungsmechanismus 40 umfasst einen Koppelmechanismus 230, der einen Schieber 232 aufweist, der mit dem Druckstück 160 koppelbar ist, das am proximalen Ende des Schubstücks 154 vorgesehen ist. Der Schieber 232 umfasst einen köcherartig gestalteten Sitz 234 und ein sich daran anschließendes Flachstück 236. Der Schieber 232 kann auf das Druckstück 160 aufgesteckt werden, um dieses in Richtung auf das distale Ende des Instruments zu drücken. Eine gegengerichtete Kraft wird durch die Feder 158 erzeugt, die in Fig. 23 angedeutet ist. Der Einbauzustand der Feder kann etwa der Darstellung in Fig. 14 entnommen werden, vgl. hierzu auch Fig. 26.

Die Handhabe 42 umfasst Betätigungsabschnitte 238, 240, die sich etwa zangenartig von proximal nach distal erstrecken. Zwischen den Betätigungsabschnitten 238, 240 erstreckt sich der Trägerschaft 228, an dessen distalem Ende die Handhabe 42 befestigt ist.

Der Betätigungsabschnitt 238 ist über ein Koppelglied 246 mit dem Flachstück 236 des köcherartigen Schiebers 232 gekoppelt. Der Betätigungsabschnitt 240 ist über ein Koppelglied 248 mit dem Flachstück 236 verbunden. Die Koppelglieder 246, 248 weisen am Flachstück 236 des Schiebers 232 den gleichen Drehpunkt auf. Wenn die beiden Betätigungsabschnitte 238, 240 der Handhabe 42 aufeinander zu bewegt werden, also zusammengedrückt werden, drängen die Koppelglieder 246, 248 den Schieber 232 gegen das Druckstück 160, um das Schubstück 154 in Richtung auf das distale Ende des Instruments 10 zu bewegen. Dies bewirkt ein Schließen der Maulteile 22, 24 des Effektors 20.

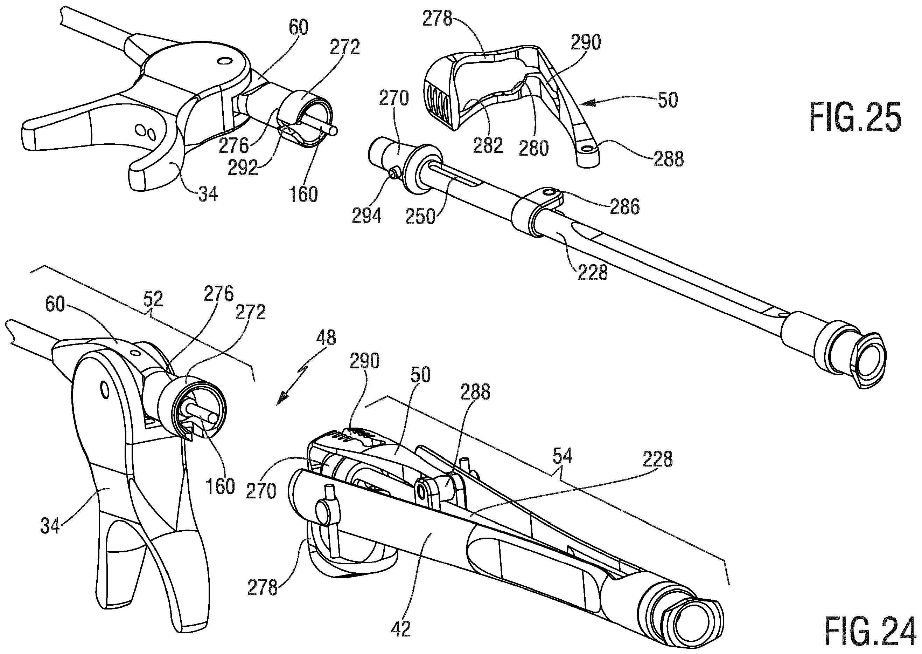

Das Koppelglied 246 ist über einen Lagerbolzen 254 gelenkig am Betätigungsabschnitt 238 aufgenommen. Das Koppelglied 248 ist über einen Lagerbolzen 256 gelenkig am Betätigungsabschnitt 240 aufgenommen. Die Koppelglieder 246, 248 erstrecken sich durch Durchtritte 250, die im Trägerschaft 228 ausgebildet sind, vgl. auch die Darstellung des Trägerschafts 228 mit dem Durchtritt 250 in Fig. 25.

Am Betätigungsabschnitt 238 ist ein Gelenkabschnitt 262 ausgebildet. Am Betätigungsabschnitt 240 ist ein Gelenkabschnitt 264 ausgebildet, vgl. Fig. 22. Die Gelenkabschnitte 262, 264 können bspw. eine Materialschwächung aufweisen, um ein stoffschlüssiges Drehgelenk und/oder einen virtuellen Drehpunkt bzw. eine virtuelle Schwenkachse für die Betätigungsabschnitte 238, 240 bereitzustellen.

Auch der Betätigungsmechanismus 40 für den Effektor 20 ist spielarm oder sogar spielfrei gestaltet. Am proximalen Ende des Schubstücks 154 ist das Druckstück 160 ausgebildet, das durch die Feder 158 in Richtung auf das proximale Ende des Instruments gedrängt wird. Vom distalen Ende her wirkt der Koppelmechanismus 230 über den Schieber 232 auf das Druckstück 160 und drängt dieses gegen die Kraft der Feder 158 in Richtung auf das proximale Ende des Instruments 10, wenn die Betätigungsabschnitte 238, 240 aufeinander zubewegt werden. Sofern auch der Koppelmechanismus 230 zumindest mit einer leichten Vorspannung auf das Druckstück 160 einwirkt, kann dieses spielarm oder spielfrei "schwimmend" zwischen dem Koppelmechanismus 230 und der Feder 158 aufgenommen sein. Zu diesem Zweck ist es vorstellbar, die Betätigungsabschnitte 238, 240 der Handhabe 42 mit einer gewissen Vorspannung zu gestalten. Mit anderen Worten kann der Koppelmechanismus 230 derart ausgelegt werden, dass auch in einem von außen unbelasteten Zustand die Betätigungsabschnitte 238, 240 nach innen drängen und somit eine Vorspannkraft auf den Schieber 232 erzeugt wird.

Mit Bezugnahme auf Fig. 24 und Fig. 25 sowie mit ergänzender Bezugnahme auf Fig. 26 und Fig. 27 wird eine beispielhafte Ausgestaltung der Schnittstelle 48 zwischen der Schaftbaugruppe 52 und dem Griffstück 54 näher veranschaulicht. Die Schnittstelle 48 kann über einen Verschlussbügel 50 verriegelt oder entriegelt werden.

Die Schnittstelle umfasst ein männliches Passteil 270 und ein weibliches Passteil 272. Das männliche Passteil 270 kann auch als Innenteil bezeichnet werden. Das weibliche Passteil 272 kann auch als Außenteil bezeichnet werden. Das männliche Passteil 270 ist in das weibliche Passteil 272 einführbar, um die Schaftbaugruppe 52 und das Griffstück 54 miteinander auszurichten. Am weiblichen Passteil 272 ist ein Halteabschnitt 276 ausgebildet, etwa in Form einer Stufe oder eines Absatzes. Der Verschlussbügel 50 weist an seinem distalen Ende einen Halteausleger 278 auf, vgl. insbesondere die Darstellung in Fig. 25, die den Verschlussbügel 50 in isolierter Form zeigt.

Über den Halteausleger 278 kann der Verschlussbügel 50 an den Halteabschnitt 276 angreifen, um den gefügten Zustand des männlichen Passteils 270 mit dem weiblichen Passteil 272 zu verriegeln.

Der Halteausleger 278 umfasst beispielhaft einen Lagesicherungsabschnitt 280, an den sich ein Freigabeabschnitt 282 anschließt, vgl. wiederum Fig. 25. Der Lagesicherungsabschnitt 280 umfasst eine Engstelle. Der Freigabeabschnitt 282 umfasst eine Aufweitung. Gemeinsam können der Lagesicherungsabschnitt 280 und der Freigabeabschnitt 282 eine Schlüssellochkontur bilden. Der Verschlussbügel 50 ist über ein Gelenk 288 an einem Lagerstück 286 schwenkbar aufgenommen. Das Lagerstück 286 ist am Trägerschaft 228 angeordnet.

Je nach aktuellem Schwenkzustand des Verschlussbügels ist der Lagesicherungsabschnitt 280 oder der Freigabeabschnitt 282 mit dem Halteabschnitt 276 ausgerichtet. Für eine Fügebewegung (bzw. eine Lösebewegung) wird der Verschlussbügel 50 derart verschwenkt, dass der Freigabeabschnitt 282 im Wesentlichen konzentrisch zum Trägerschaft 228 bzw. zum männlichen Passteil 270 ausgerichtet ist. Sodann kann das männliche Passteil 270 mit dem weiblichen Passteil 272 gefügt werden, vgl. auch den Zustand in Fig. 26.

Hiernach kann der Verschlussbügel 50 derart verschwenkt werden, dass der Lagesicherungsabschnitt 280 an den Halteabschnitt 276 angreift, um den Fügezustand zwischen dem männlichen Passteil 270 und dem weiblichen Passteil 272 zu sichern. Zum Entriegeln der Schnittstelle 48 kann eine gegensinnige Bewegung auf den Verschlussbügel 50 aufgebracht werden, um den Lagesicherungsabschnitt 280 aus dem verrasteten Zustand am Halteabschnitt 276 herauszuführen. Es ist vorstellbar, den Verschlussbügel 50 derart am Trägerschaft 228 aufzunehmen, dass sich eine Vorspannung in Richtung auf den verriegelten Zustand ergibt. Dies kann die Sicherheit zusätzlich erhöhen. Die Gefahr von Fehlbedienung kann verringert werden.

Beispielhaft ist der Lagesicherungsabschnitt 280 am Halteausleger 278 des Verschlussbügels 50 derart gestaltet, dass ein Ausrücken vom Halteabschnitt 276 nur durch eine elastische Verformung, etwa durch ein seitliches Aufweiten, des Verschlussbügels 50 im Bereich des Halteauslegers 278 möglich ist. Auf diese Weise muss eine bestimmte Kraft aufgebracht werden, um den Lagesicherungsabschnitt 280 auszurasten und den Freigabeabschnitt 282, der einen größeren Querschnitt aufweist, mit dem Halteabschnitt 276 in Überdeckung zu bringen. Ferner ist es vorstellbar, an einem Rücken des Verschlussbügels 50 eine Ausnehmung 290 auszubilden, die in den Lagesicherungsabschnitt 280 mündet. Auf diese Weise ist eine definierte Verformbarkeit des Halteauslegers 278 im Bereich des Lagesicherungsabschnitts 280 gegeben.

Zur Sicherung einer relativen Drehlage zwischen der Schaftbaugruppe 52 und dem Griffstück 54 ist beispielhaft eine Nut 292 am weiblichen Passteil 272 ausgebildet. Die Nut 292 erstreckt sich axial und ist an ihrem proximalen Ende offen. Am männlichen Passteil 270 ist ein Führungsstift 294 angeordnet, der radial zu einer Längsachse des Instruments 10 orientiert ist. Beim Fügen des männlichen Passteils 270 mit dem weiblichen Passteil 272 kann der Führungsstift 294 in die Nut 292 eingreifen, um die gewünschte Drehlage zu sichern.

Die geschnittenen Darstellungen in Fig. 26 und Fig. 27 veranschaulichen, dass am männlichen Passteil 270 eine Konusfläche 300 ausgebildet ist, an die sich eine Zylinderfläche 304 anschließt. Am weiblichen Passteil 272 ist eine Konusfläche 302 ausgebildet, an die sich eine Zylinderfläche 306 anschließt. Die Konusfläche 302 und die Zylinderfläche 306 sind Innenflächen. Die Konusfläche 300 und die Zylinderfläche 304 sind Außenflächen. Im gefügten Zustand ergibt sich eine definierte axiale und konzentrische Ausrichtung zwischen der Schaftbaugruppe 52 und dem Griffstück 54, die spielarm oder sogar spielfrei ist. Eine relative Drehlage zwischen der Schaftbaugruppe 52 und dem Griffstück 54 wird durch die Nut 292 und den Führungsstift 294 definiert.

Er ist jedoch auch vorstellbar, auf die Konusflächen 300, 302 zu verzichten. Demgemäß können die Passteile 270, 272 primär mit den Zylinderflächen 304, 306 versehen sein, die eine konzentrische Auslegung sicherstellen.

Fig. 26 und Fig. 27 veranschaulichen ferner, dass die Schnittstelle 48 auch das Druckstück 160 auf der Seite der Schaftbaugruppe 52 sowie den Schieber 232 auf der Seite des Griffstücks 54 umfasst. Über den Schieber 232 kann eine Schiebebewegung auf das Druckstück 160 übertragen werden, um das Schubstück 154 in Richtung auf das distale Ende des Instruments zu verlagern, um den Effektor 20 zu steuern. Über den Schieber 232 werden vorrangig Druckkräfte auf das Druckstück 160 übertragen.

Demgemäß kann in einfacher Weise durch Aufstecken des Schiebers 232 auf das Druckstück 160 eine Verbindung erzeugt werden. Eine Rückstellbewegung bzw. Rückstellkraft wird durch die Feder 158 erzeugt, die zwischen dem Druckstück 160 und dem Lagerstück 60 angeordnet ist, wobei die Feder 158 konzentrisch zur Längsachse des Instruments 10 ausgerichtet ist. Somit ist auch die Bewegungsübertragung für den Bewegungsfreiheitsgrad des Effektors 20 spielarm oder sogar spielfrei gestaltet.

## Patentansprüche

1. Chirurgisches Instrument, insbesondere Instrument für die Neurochirurgie, das Folgendes aufweist:
- einen Schaft (12), wobei am Schaft (12) ein Gelenkabschnitt (16) ausgebildet ist,
- ein Lagerstück (60) mit einem sich von einem distalen zu einem proximalen Ende erstreckenden Durchgang (150) zur Aufnahme eines zumindest proximalen Abschnittes des Schafts (12),
- einen proximalen Handhabungsabschnitt (30) an einem proximalen Ende des Schaftes (12),
- einen distalen Effektor (20) an einem distalen Ende des Schaftes (12), und
- eine Schnittstelle (48), an der das Instrument in einen distalen und einen proximalen Teil zerlegbar ist, wobei das Instrument an der Schnittstelle (48) in eine distale Schaftbaugruppe (52) und ein proximales Griffstück (54) zerlegbar ist,
wobei der Effektor (20) über einen Betätigungsmechanismus (40) gesteuert wird, der ein Schubstück (154) aufweist, und
wobei der Effektor (20) ein erstes Maulteil (22) und ein zweites Maulteil (24) aufweist, die relativ zueinander verschwenkbar sind, und
wobei das Schubstück (154) nach distal verfahrbar ist, um das erste Maulteil (22) und das zweite Maulteil (24) aufeinander zu zu bewegen und zu schließen **dadurch gekennzeichnet, dass**
die Schnittstelle (48) auf der Seite der Schaftbaugruppe (52) ein Druckstück (160) sowie auf der Seite des Griffstücks (54) einen Schieber (232) umfasst, der auf das Druckstück (160) aufsteckbar ist, das mit dem Schieber (232) zur Krafteinleitung in das Druckstück (160) koppelbar ist,
das Druckstück (160) am proximalen Ende des Schubstücks (154) vorgesehen ist, wobei das Schubstück (154) durch eine Feder (158) nach proximal vorgespannt ist, die sich zwischen dem Druckstück (160) und einem distalen Anschlag (162) am Lagerstück (60) erstreckt, um das Schubstück (154) in Richtung auf das proximale Ende zu drängen, und
der Schieber (232) dazu ausgebildet ist, eine Schiebebewegung auf das Druckstück (160) zu übertragen, um das Schubstück (154) in Richtung auf das distale Ende des Instruments zu verlagern.

2. Instrument nach Anspruch 1, wobei der Gelenkabschnitt (16) ausgehend von einer Mittelposition einseitig auslenkbar ist, und wobei das Schubstück (154) als Schubstange oder Schubdraht ausgebildet ist.

3. Instrument nach Anspruch 1 oder 2, wobei die Schnittstelle (48) Passteile (270, 272) umfasst, die ein männliches Teil (270) und ein weibliches Teil (272) umfassen, die miteinander koppelbar sind.

4. Instrument nach einem der Ansprüche 1 bis 3, wobei der Schnittstelle (48) ein Verschlussbügel (50) zugeordnet ist, der dazu ausgebildet ist, an einen Halteabschnitt (276) anzugreifen, um eine axiale Relativlage zwischen der Schaftbaugruppe (52) und dem Griffstück (54) zu sichern, wobei der Verschlussbügel (50) gelenkig am Griffstück (54) aufgenommen ist, und wobei der Halteabschnitt (276) vorzugsweise bei der Schaftbaugruppe (52) ausgebildet ist.

5. Instrument nach Anspruch 4, wobei am Verschlussbügel (50) ein Halteausleger (278) ausgebildet ist, der den Halteabschnitt (276) zumindest abschnittsweise umgibt, wobei der Halteausleger (278) einen Lagesicherungsabschnitt (280) und einen aufgeweiteten Freigabeabschnitt (282) umfasst, und wobei der Halteausleger (278) vorzugsweise an einem distalen Ende des Verschlussbügels (50) ausgebildet ist.

6. Instrument nach einem der vorhergehenden Ansprüche, wobei die Schnittstelle (48) ferner eine Drehlagensicherung aufweist, insbesondere in Form eines Führungsstiftes (294), der in eine sich axial erstreckende Nut (292) eingreift.

7. Instrument nach einem der Ansprüche 1 bis 6, wobei das Schubstück (154) an seinem distalseitigen Ende mit einen Koppelstück (174) verbunden ist, das einen ersten Mitnehmer (178) für das erste Maulteil (22) und einen zweiten Mitnehmer (180) für das zweite Maulteil (24) aufweist, wobei das erste Maulteil (22) eine Mitnahmeausnehmung (186) aufweist, wobei das zweite Maulteil (24) eine Mitnahmeausnehmung (188) aufweist, wobei der erste Mitnehmer (178) des Koppelstücks (174) in die Mitnahmeausnehmung (186) des ersten Maulteils (22) eingreift, und wobei der zweite Mitnehmer (180) des Koppelstücks (174) in die Mitnahmeausnehmung (188) des zweiten Maulteils (24) eingreift.

8. Instrument nach einem der vorhergehenden Ansprüche, ferner aufweisend ein Kopfstück (166) am distalen Ende des Schaftes (12), wobei das erste Maulteil (22) schwenkbar am Kopfstück (166) gelagert ist, wobei das zweite Maulteil (24) schwenkbar am Kopfstück (166) gelagert ist, und wobei Schwenkachsen des ersten Maulteils (22) und des zweiten Maulteils (24) parallel zueinander und voneinander beabstandet sind.

9. Instrument nach Anspruch 8, wobei das erste Maulteil (22) eine Führungsbahn (206) aufweist, wobei das zweite Maulteil (24) eine Führungsbahn (208) aufweist, und wobei ein am Kopfstück (166) aufgenommener Führungsstift (214) in der Führungsbahn (206) des ersten Maulteils (22) und der Führungsbahn (208) des zweiten Maulteils (24) aufgenommen ist.

10. Instrument nach Anspruch 9, sofern auf Anspruch 7 rückbezogen, wobei sich das Koppelstück (174) zumindest abschnittsweise zwischen dem ersten Maulteil (22) und dem zweiten Maulteil (24) erstreckt, und wobei der Führungsstift (214) eine Ausnehmung (216) des Koppelstücks (174) durchragt.

11. Instrument nach einem der vorhergehenden Ansprüche, wobei der Schieber (232) längsverschieblich am Handhabungsabschnitt (30) gelagert ist, und wobei der Schieber (232) mit einer Handhabe (42) gekoppelt ist, die zumindest einen Betätigungsabschnitt (238, 240) aufweist, der schwenkbar am Handhabungsabschnitt (30) aufgenommen ist.

12. Instrument nach Anspruch 11, wobei am Handhabungsabschnitt (30) ein Koppelmechanismus (230) ausgebildet ist, der die Handhabe (42), den Schieber (232) und zumindest ein Koppelglied (246, 248) umfasst, wobei eine Schwenkbewegung des zumindest einen Betätigungsabschnitts (238, 240) in eine Schubbewegung des Schiebers (232) überführt wird.

13. Instrument nach Anspruch 11 oder 12, sofern auf Anspruch 4 rückbezogen, wobei die Handhabe (42) zangenartig gestaltet ist und zwei Arme (44, 46) aufweist, die den Betätigungsabschnitten (238, 240) zugeordnet sind, und die in Richtung auf das proximale Ende des Schaftes (12) geöffnet sind, und wobei die Arme (44, 46) den Verschlussbügel (50) und einen Steuerhebel (34) zumindest abschnittsweise seitlich umgreifen.

14. Instrument nach einem der Ansprüche 11 bis 13, sofern auf Anspruch 4 rückbezogen, wobei entlang einer Haupterstreckungsrichtung des Instruments (10), von proximal nach distal gesehen, die Handhabe (42), der Verschlussbügel (50) und der oder ein Steuerhebel (34) nacheinander gelenkig aufgenommen sind, wobei Schwenkachsen des Verschlussbügels (50) und des Steuerhebels (34) parallel zueinander sind, und wobei eine Schwenkbewegung der Handhabe (42) um eine Schwenkachse erfolgt, die senkrecht zu den Schwenkachsen des Verschlussbügels (50) und des Steuerhebels (34) ist.

15. Instrument nach Anspruch 14, wobei die Handhabe (42) und der Verschlussbügel (50) gelenkig an einem Trägerschaft (228) des Griffstücks (54) aufgenommen sind.

## Claims

1. Surgical instrument, in particular an instrument for neurosurgery, comprising:
- a shaft (12) wherein a joint section (16) is formed on the shaft (12),
- a support piece (60) with a passage (150) extending from a distal to a proximal end for receiving at least a proximal portion of the shaft (12),
- a proximal handling portion (30) at a proximal end of the shaft (12),
- a distal effector (20) at a distal end of the shaft (12), and
- an interface (48) at which the instrument can be disassembled into a distal and a proximal part, wherein the instrument can be disassembled at the interface (48) into a distal shaft assembly (52) and a proximal handle (54),
wherein the effector (20) is controlled by an actuating mechanism (40) which includes a push piece (154), and
wherein the effector (20) includes a first jaw part (22) and a second jaw part (24) which are pivotable relative to each other,
wherein the push piece (154) is movable distally to move the first jaw part (22) and the second jaw part (24) towards each other and to close them,
**characterized in that**
the interface (48) comprises on the side of the shaft assembly (52) a pressure piece (160) and on the side of the handle (54) a slider (232) which can be plugged onto the pressure piece (160) which can be coupled with the slider (232) for force transmission into the pressure piece (160),
the pressure piece (160) is provided at the proximal end of the push piece (154), wherein the push piece (154) is pre-tensioned proximally by a spring (158) extending between the pressure piece (160) and a distal stop (162) on the support piece (60) to urge the push piece (154) towards the proximal end, and
the slider (232) is arranged to transmit a sliding movement to the pressure piece (160) to move the push piece (154) towards the distal end of the instrument.

2. Instrument according to claim 1, wherein the joint section (16) is deflectable to one side starting from a middle position, and wherein the push piece (154) is arranged as a push rod or push wire.

3. Instrument according to claim 1 or 2, wherein the interface (48) comprises fitting parts (270, 272) including a male part (270) and a female part (272) which are engageable with each other.

4. Instrument according to any one of claims 1 to 3, wherein the interface (48) is assigned a locking bracket (50) arranged to engage a retaining section (276) to secure an axial relative position between the shaft assembly (52) and the handle (54), wherein the locking bracket (50) is pivotally mounted on the handle (54), and wherein the retaining section (276) is preferably formed on the shaft assembly (52).

5. Instrument according to claim 4, wherein a retaining arm (278) is formed at the locking bracket (50), which at least partially surrounds the retaining section (276), wherein the retaining arm (278) includes a position securing section (280) and a widened release section (282), and wherein the retaining arm (278) is preferably formed at a distal end of the locking bracket (50).

6. Instrument according to any of the preceding claims, wherein the interface (48) further includes a rotational position securing feature, particularly in the form of a guide pin (294) which engages in an axially extending groove (292).

7. Instrument according to any one of claims 1 to 6, wherein the push piece (154) is connected at its distal end to a coupling piece (174) which includes a first driver (178) for the first jaw part (22) and a second driver (180) for the second jaw part (24), wherein the first jaw part (22) includes a driving recess (186), and wherein the second jaw part (24) includes a driving recess (188), wherein the first driver (178) of the coupling piece (174) engages in the driving recess (186) of the first jaw part (22), and wherein the second driver (180) of the coupling piece (174) engages in the driving recess (188) of the second jaw part (24).

8. Instrument according to any one of the preceding claims, further comprising a head piece (166) at the distal end of the shaft (12), wherein the first jaw part (22) is pivotally mounted on the head piece (166), wherein the second jaw part (24) is pivotally mounted on the head piece (166), and wherein pivot axes of the first jaw part (22) and the second jaw part (24) are parallel to each other and spaced apart from one another.

9. Instrument according to claim 8, wherein the first jaw part (22) includes a guideway (206), wherein the second jaw part (24) includes a guideway (208), and wherein a guide pin (214) mounted on the head piece (166) is accommodated in the guideway (206) of the first jaw part (22) and the guideway (208) of the second jaw part (24).

10. Instrument according to claim 9, if referring back to claim 7, wherein the coupling piece (174) extends at least partially between the first jaw part (22) and the second jaw part (24), and wherein the guide pin (214) extends through a recess (216) of the coupling piece (174).

11. Instrument according to any one of the preceding claims, wherein the slider (232) is longitudinally displaceable on the handling portion (30), and wherein the slider (232) is coupled to a handle (42) which includes at least one actuating section (238, 240) which is pivotally mounted on the handling portion (30).

12. Instrument according to claim 11, wherein a coupling mechanism (230) is formed on the handling portion (30) comprising the handle (42), the slider (232), and at least one coupling member (246, 248), wherein a pivoting movement of the at least one actuating section (238, 240) is converted into a pushing movement of the slider (232).

13. Instrument according to claim 11 or 12, if referring back to claim 4, wherein the handle (42) is arranged forceps-like and includes two arms (44, 46) associated with the actuating sections (238, 240) and which are opened towards the proximal end of the shaft (12), and wherein the arms (44, 46) at least partially surround the locking bracket (50) and a control lever (34) laterally.

14. Instrument according to any one of claims 11 to 13, if referring back to claim 4, wherein along a main extension direction of the instrument (10) viewed from proximal to distal, the handle (42), the locking bracket (50), and the or a control lever (34) are successively pivotally mounted, wherein pivot axes of the locking bracket (50) and the control lever (34) are parallel to each other, and wherein a pivoting movement of the handle (42) takes place about a pivot axis perpendicular to the pivot axes of the locking bracket (50) and the control lever (34).

15. Instrument according to claim 14, wherein the handle (42) and the locking bracket (50) are pivotally mounted on a carrier shaft (228) of the handle (54).

## Revendications

1. Instrument chirurgical, en particulier instrument pour la neurochirurgie, qui présente les éléments suivants :
- une tige (12), dans lequel une section d'articulation (16) est réalisée sur la tige (12),
- une pièce de support (60) comportant un passage (150) s'étendant d'une extrémité distale à une extrémité proximale pour la réception d'une section au moins proximale de la tige (12),
- une section de manipulation (30) proximale à une extrémité proximale de la tige (12),
- un effecteur (20) distal à une extrémité distale de la tige (12), et
- une interface (48) au niveau de laquelle l'instrument peut être désassemblé en une partie distale et en une partie proximale, dans lequel l'instrument peut être désassemblé au niveau de l'interface (48) en un ensemble tige (52) distal et en une pièce de préhension (54) proximale,
dans lequel l'effecteur (20) est commandé par l'intermédiaire d'un mécanisme d'actionnement (40) qui présente une pièce de poussée (154), et
dans lequel l'effecteur (20) présente une première partie de mâchoire (22) et une seconde partie de mâchoire (24) qui peuvent pivoter l'une par rapport à l'autre, et dans lequel la pièce de poussée (154) peut être déplacée vers le côté distal pour déplacer la première partie de mâchoire (22) et la seconde partie de mâchoire (24) l'une vers l'autre et les fermer
**caractérisé en ce que**
l'interface (48) comprend, du côté de l'ensemble tige (52), une pièce de pression (160) ainsi que, du côté de la pièce de préhension (54), un coulisseau (232) qui peut être enfiché sur la pièce de pression (160), laquelle peut être accouplée au coulisseau (232) pour l'introduction d'une force dans la pièce de pression (160),
la pièce de pression (160) est prévue à l'extrémité proximale de la pièce de poussée (154), dans lequel la pièce de poussée (154) est précontrainte vers le côté proximal par un ressort (158) qui s'étend entre la pièce de pression (160) et une butée distale (162) au niveau de la pièce de support (60) afin de pousser la pièce de poussée (154) en direction de l'extrémité proximale, et
le coulisseau (232) est réalisé pour transmettre un mouvement de coulissement à la pièce de pression (160) afin de déplacer la pièce de poussée (154) en direction de l'extrémité distale de l'instrument.

2. Instrument selon la revendication 1, dans lequel la section d'articulation (16) peut être déviée d'un côté à partir d'une position centrale, et dans lequel la pièce de poussée (154) est réalisée en tant que tige de poussée ou fil de poussée.

3. Instrument selon la revendication 1 ou 2, dans lequel l'interface (48) comprend des parties d'ajustement (270, 272) qui comprennent une partie mâle (270) et une partie femelle (272) qui peuvent être accouplées l'une à l'autre.

4. Instrument selon l'une des revendications 1 à 3, dans lequel l'interface (48) est associée à un étrier de verrouillage (50) qui est réalisé pour venir en prise avec une section de retenue (276) afin d'assurer une position relative axiale entre l'ensemble tige (52) et la pièce de préhension (54), dans lequel l'étrier de verrouillage (50) est reçu de manière articulée sur la pièce de préhension (54), et dans lequel la section de retenue (276) est de préférence réalisée au niveau de l'ensemble tige (52).

5. Instrument selon la revendication 4, dans lequel une branche de retenue (278) est réalisée sur l'étrier de fermeture (50), laquelle entoure au moins dans certaines sections la section de retenue (276), dans lequel la branche de retenue (278) comprend une section de blocage de position (280) et une section de libération (282) élargie, et dans lequel la branche de retenue (278) est réalisée de préférence à une extrémité distale de l'étrier de fermeture (50).

6. Instrument selon l'une des revendications précédentes, dans lequel l'interface (48) présente en outre un dispositif de blocage de position antirotation, en particulier sous la forme d'une goupille de guidage (294) qui vient en prise dans une rainure (292) s'étendant axialement.

7. Instrument selon l'une des revendications 1 à 6, dans lequel la pièce de poussée (154) est reliée à son extrémité distale à une pièce d'accouplement (174) qui présente un premier moyen d'entraînement (178) pour la première partie de mâchoire (22) et un second moyen d'entraînement (180) pour la seconde partie de mâchoire (24), dans lequel la première partie de mâchoire (22) présente un évidement d'entraînement (186), dans lequel la seconde partie de mâchoire (24) présente un évidement d'entraînement (188), dans lequel le premier moyen d'entraînement (178) de la pièce d'accouplement (174) vient en prise dans l'évidement d'entraînement (186) de la première partie de mâchoire (22), et dans lequel le second moyen d'entraînement (180) de la pièce d'accouplement (174) vient en prise dans l'évidement d'entraînement (188) de la seconde partie de mâchoire (24).

8. Instrument selon l'une des revendications précédentes, présentant en outre une pièce de tête (166) à l'extrémité distale de la tige (12), dans lequel la première partie de mâchoire (22) est supportée de manière à pouvoir pivoter sur la pièce de tête (166), dans lequel la seconde partie de mâchoire (24) est supportée de manière à pouvoir pivoter sur la pièce de tête (166), et dans lequel des axes de pivotement de la première partie de mâchoire (22) et de la seconde partie de mâchoire (24) sont parallèles entre eux et espacés l'un de l'autre.

9. Instrument selon la revendication 8, dans lequel la première partie de mâchoire (22) présente une voie de guidage (206), dans lequel la seconde partie de mâchoire (24) présente une voie de guidage (208), et dans lequel une goupille de guidage (214) reçue sur la pièce de tête (166) est reçue dans la voie de guidage (206) de la première partie de mâchoire (22) et dans la voie de guidage (208) de la seconde partie de mâchoire (24).

10. Instrument selon la revendication 9 lorsqu'elle dépend de la revendication 7, dans lequel la pièce d'accouplement (174) s'étend au moins dans certaines sections entre la première partie de mâchoire (22) et la seconde partie de mâchoire (24), et dans lequel la goupille de guidage (214) traverse un évidement (216) de la pièce d'accouplement (174).

11. Instrument selon l'une des revendications précédentes, dans lequel le coulisseau (232) est supporté de manière à pouvoir coulisser longitudinalement sur la section de manipulation (30), et dans lequel le coulisseau (232) est accouplé à une poignée (42) qui présente au moins une section d'actionnement (238, 240) reçue de manière à pouvoir pivoter sur la section de manipulation (30).

12. Instrument selon la revendication 11, dans lequel un mécanisme d'accouplement (230) est réalisé sur la section de manipulation (30), lequel comprend la poignée (42), le coulisseau (232) et au moins un élément d'accouplement (246, 248), dans lequel un mouvement de pivotement de l'au moins une section d'actionnement (238, 240) est converti en un mouvement de poussée du coulisseau (232).

13. Instrument selon la revendication 11 ou 12 lorsqu'elle dépend de la revendication 4, dans lequel la poignée (42) est conçue en forme de pince et présente deux bras (44, 46) qui sont associés aux sections d'actionnement (238, 240) et qui sont ouverts en direction de l'extrémité proximale de la tige (12), et dans lequel les bras (44, 46) entourent latéralement, au moins dans certaines sections, l'étrier de fermeture (50) et un levier de commande (34).

14. Instrument selon l'une des revendications 11 à 13 lorsqu'elle dépend de la revendication 4, dans lequel, le long d'une direction d'extension principale de l'instrument (10), vus du côté proximal vers le côté distal, la poignée (42), l'étrier de fermeture (50) et le ou un levier de commande (34) sont reçus successivement de manière articulée, dans lequel des axes de pivotement de l'étrier de fermeture (50) et du levier de commande (34) sont parallèles entre eux, et dans lequel un mouvement de pivotement de la poignée (42) est effectué autour d'un axe de pivotement qui est perpendiculaire aux axes de pivotement de l'étrier de fermeture (50) et du levier de commande (34).

15. Instrument selon la revendication 14, dans lequel la poignée (42) et l'étrier de fermeture (50) sont reçus de manière articulée sur une tige de soutien (228) de la pièce de préhension (54).
